(19)

Europäisches
Patentamt
European
Patent Office
Office européen
des brevets

(11) **EP 2 407 555 A1**

(12) **EUROPEAN PATENT APPLICATION**

(43) Date of publication:
**18.01.2012 Bulletin 2012/03**

(51) Int Cl.:
***C12Q 1/68*** *(2006.01)*

(21) Application number: **10382194.8**

(22) Date of filing: **14.07.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Fundació Institut de Recerca Hospital
Universitari
Vall d'Hebron, Fundació Privada
08035 Barcelona (ES)**

(72) Inventors:
• **Doll, Andreas
08035 Barcelona (ES)**

• **Rigau Resina, Marina
08035 Barcelona (ES)**
• **Morote Robles, Juan
08035 Barcelona (ES)**
• **Abal Posada, Miguel
08035 Barcelona (ES)**
• **Reventós Puigjaner, Jaume
08035 Barcelona (ES)**

(74) Representative: **ABG Patentes, S.L.
Avenida de Burgos 16D
Edificio Euromor
28036 Madrid (ES)**

(54) **Methods and kits for the diagnosis of prostate cancer**

(57)    The invention relates to methods and kits for the diagnosis of prostate cancer in a subject, for assessing or monitoring the response to a therapy in a subject having prostate cancer (PCa) or for monitoring the progression of prostate cancer (PCa) based on the detection of alteration in the expression levels of at least one gene selected from the group of PCA3, PSMA and PSGR. The invention relates as well to methods for assessing whether a subject has to be subjected to a prostate biopsy, said subject having a serum PSA range within 4-10 ng/mL.

EP 2 407 555 A1

**Description**

**FIELD OF THE INVENTION**

The invention fall within the field of diagnosis and, more specifically, in the field of diagnosis of prostate cancer by means of using a combination of genes the expression of which is over-expressed in prostate cancer cells as compared to their expression in normal (i.e., non-cancer) prostate cells.

**BACKGROUND OF THE INVENTION**

Prostate cancer (PCa) has converted into the most common type of cancer in the Western male population, where it is responsible for more male deaths than any other cancer, except lung cancer. The risk to develop this cancer during lifetime is estimated to 1 in 6 men and the risk of death due to metastatic prostate cancer is 1 in 30. Although the introduction of the prostate-specific antigen (PSA) test in the late 80s of the past century has led to a dramatic increase in PCa detection, the mortality has not decreased significantly [Jemal, A., et al., (2007). Cancer statistics, 2007. CA: a cancer journal for clinicians, 2009, June 9, 57, 43-66]. There are two main reasons: a missing, no invasive, early diagnosis method accepted by a wide range of the male population over 50 years old and the fact that only if the disease is still confined to the prostate it can be cured by radical surgery or radiation therapy.

Since there are no symptoms in the early stages, less than 30% of the males, in contrast to more than 70% of the female population, undergo the necessary preventive exams. Currently, these exams consist in a diagnostic tripod: (i) the quantification of PSA in the brood of the patient, (ii) a digital rectal massage (DRM) examination, and, finally, (iii) the confirmation by a diagnostic set of about 10 transrectal ultrasound guided needle biopsy (TRUS) which are performed in men with an abnormal DRM, an elevated PSA (>4.0 ng/mL), with an actual tendency to >2.5 ng/mL, or PSA velocity (rate of PSA change) >0.4 to 0.75 ng/mL/year. Said exams constitute the gold standard of care for PCa screening in the western hemisphere. Nevertheless, PSA and DRM lack significantly in specificity and biopsy lacks ideal sensitivity (12-30%) false negatives). One of the limitations of PSA as a tumor marker is its lack of specificity, which results in a high negative biopsy rate. At the gray zone of PSA (4-10 ng/mL) a substantial overlap in serum PSA values exist between men with early PCa and benign conditions of the prostate such as benign hyperplasia (BPH) and asymptomatic chronic prostatitis [Loeb, S. et al., (2009). Exclusion of inflammation in the differential diagnosis of an elevated prostate-specific antigen (PSA). Urol Oncol 27, 64-66; Pannek, J., and Partin, A.W. (1997). Prostate-specific antigen: what's new in 1997. Oncology (Williston Park) 11, 1273-1278; discussion 1279-1282].

Various attempts have been made to overcome the limitations of PSA screening. These include the use of age-adjusted PSA cut-off points, free PSA, PSA density, PSA velocity, PSA slope and PSA doubling time. All have been proposed as a means to improve the detection of "clinically important" PCa cases. Nevertheless, no evidence has suggested that any of these testing strategies improves health outcomes.

As a consequence of the current screening parameters around 2/3 of the approximately 390,000 biopsies made yearly in Europe are unnecessary [Catalona, WJ. et al., Measurement of prostate-specific antigen in serum as a screening test for prostate cancer. N Engl J Med 324:1156-61, (1991); Makinen, T. et al. Second round results of the Finnish population-based prostate cancer screening trial. Clin Cancer Res 10:2231-6 (2004)]. The false positive rate of a biopsy is more or less zero but the false negative rate in the first biopsy oscillates around 12-30%. Consequently, many men with negative biopsy findings undergo repeat biopsies to rule out PCa.

More accurate tests are needed that can help to identify which patients are at high risk of developing PCa, and from whom repeat prostate biopsies are mandatory. Consequently, there is an urgent need to detect PCa at an early stage using non-invasive procedures.

A wide range of promising PCa biomarkers that are not only prostate-specific, but are also over-expressed in prostate tumors have been identified, including CpG hypermethylation of GSTP1, the TMPRSS2:ERG gene fusions and RNA biomarker PCA3 [Marks, L.S., and Bostwick, D.G. (2008). Prostate Cancer Specificity of PCA3 Gene Testing: Examples from Clinical Practice. Rev Urol 10, 175-181; Saramaki et al. (2008). TMPRSS2:ERG fusion identifies a subgroup of prostate cancers with a favorable prognosis. Clin Cancer Res 14, 3395-3400; Vener et al. (2008). Development of a multiplexed urine assay for prostate cancer diagnosis. Clin Chem 54, 874-882]. As prostate cells can be detected in the urine of men with PCa, urine-based diagnostic tests have the advantage of being non- or minimal invasive and therefore will be accepted by a wider range of the male population. Although urine-based testing for PCA3 expression and GSTP1 methylation has already been documented in large screening programs, there are only two studies, one using RNA and the other using genomic DNA, based on a diagnostic profile that take into account the heterogeneity of cancer development [Laxman et al. (2008). Cancer Res 68, 645-649].

WO 03/009814 discloses a method of assessing whether a patient is afflicted with PCa which comprises comparing the level of expression of a marker selected from a set of markers in a patient sample and the normal level of expression of said marker in a control non-prostate cancer sample, wherein a significant increase in the level of expression of said marker in the patient sample and the normal level is an indication that the patient is afflicted with PCa. PCA3 ((M381), PSMA (FOLH1) and PSGR (M311) markers are included in said set of markers; nevertheless, WO 03/009814 does not disclose the specific combination of said PCA3, PSMA and PSGR markers as a specific three-gene panel for the early detection of PCa.

DE 10 2006 032 394 discloses a method for diagnosing PCa which comprises comparing the expression of at least two markers selected from the group of markers ZIP/CREB3L4, AMACR, DD3/PCA3, D-GPCR, EZH2, PCGEM1, PDEF, prostein, PSGR/OR51E2, PSMA/FOLH1, TMPRSS2 and TRPM8 in a patient sample; however, none of the preferred combinations of markers mentioned in DE 10 2006 032 394 consists in the specific combination of PCA3, PSMA and PSGR markers.

Further, WO 2008/121132 discloses a method of assessing whether a patient is afflicted with PCa which comprises comparing the level of expression of a marker selected from a set of markers in a patient sample with a reference value. PCA3, PSMA and PSGR markers are included in said set of markers; nevertheless, WO 2008/121132 does not disclose the specific combination of said PCA3, PSMA and PSGR markers as a specific three-gene panel for the early detection of PCa.

Despite the fact that PSA serum measurement in combination with a DRM and TRUS constitute the gold standard of care for PCa screening in the western hemisphere, PSA and DRM lack significantly in specificity and biopsy lacks ideal sensitivity (12-30% false negatives).

One of the most commonly used markers for the diagnosis of prostate cancer is determination of PCA3 in tissue samples. Van Gils *et al.* [van Gils MP, et al.. Clin Cancer Res 13:939-43 (2007)] reported a PCA3 sensitivity of 65% and specificity of 66% (AUC 0.66) in 534 patients (serum PSA between 3 and 15 ng/mL), including 174 men (33%) with cancer-positive biopsies. Hessels *et al.* [Hessels D, et al. Eur Urol 44:8-15; discussion 15-6 (2003)] observed a sensitivity of 67% and specificity of 83% (AUC 0.72) in a population study of 108 patients. Marks *et al.* [Marks LS, et al.; Urology 69:532-5 (2007)] observed a sensitivity of 58% and specificity of 72% (AUC 0.68) in a population study of 233 patients (PSA≥2.5 ng/L) with cancer representation at 27% after repeat biopsy. Groskopf *et al.* [Groskopf J, et al.;. Clin Chem 52:1089-95 (2006)] observed a sensitivity of 69% and specificity of 79% (AUC 0.75) in a population study of 70 patients. Haese *et al.* [Haese A, et al.; Eur Urol 54:1081-8 (2008)] reported a PCA3 sensitivity of 47% and specificity of 72% (AUC 0.66) with a cut-off of 35 in 463 patients in a study to identify patients with a high risk of a positive repeat biopsy.

Further, despite the research carried out in this topic, there are currently very few tumor markers which are useful from the clinical point of view both for the diagnosis of PCa. In addition, a very high number of unnecessary biopsies (around 260,000) are made yearly in Europe. Therefore, there is a need in the art for methods which allow the diagnosis of PCa which solve any of the above mentioned drawbacks. Said methods, advantageously, should be able to detect PCa at an early stage using non-invasive procedures. Consequently, additional biomarkers are needed to supplement or potentially replace the currently used diagnostic techniques.

## SUMMARY OF THE INVENTION

A novel multiplex panel of urine transcripts that outperforms PCA3 transcript alone for the detection of PCa as well as PSA alone has been identified. Inventors have tested urine sediments after prostate massage for over-expression of the putative prostate cancer biomarkers PCA3, PSGR, and PSMA from 154 patients presenting for prostate biopsy. The area under the curve (AUC) for the combined marker model was 0.80. The PCa detection rate by a prostate biopsy was 37% (57/154). Subsequently, inventors tested specifically its clinical usefulness in the target subset of 77 men with serum PSA between 4 and 10 ng/mL and no previous biopsy (special interest group). PSA density (PSAD) was also included in this analysis as a classical clinical tool to increase PSA specificity. Using a multiplex model the area under the curve (AUC) in men within said group of special interest was 0.89 and in the overall group 0.80. Having a sensitivity of 96% the specificity was 62% (40% overall group). Using said 3-gene combination with PSAD would allow saving 42% (26% overall group) of unnecessary performed biopsies. Thus, inventors have demonstrated a sensitive method to suspect PCa in urine which can be used to increase the specificity of PSA avoiding a significant number of unnecessary biopsies. This novel method increases the diagnostic efficiency of PSA and it is especially useful in the gray zone of PSA.

Thus, the invention is in based on the identification of a combination of genes whose differential expression is associated with PCa. A further use of this model is to assess whether a biopsy should be performed or not. In various aspects, the invention provides methods of evaluating the presence or absence (e.g., diagnosing or prognosing) of PCa, as well as methods of assessing or monitoring the response to therapy in a subject having PCa, and methods of monitoring the progression of PCa in a subject, said methods being based on a sample from the subject and determining a quantitative measure of the amount of 3 specific genes (PCA3, PSMA and PSGR) associated to PCa.

Thus, in a first aspect, the invention relates to a method for the diagnosis of prostate cancer (PCa) with a desired sensitivity in a subject which comprises

(i) determining the expression levels of the PCA3, PSMA and PSGR genes in a sample isolated from said subject,

(ii) comparing the expression levels of said PCA3, PSMA and PSGR genes with predetermined cut off values for each of said genes wherein said predetermined cut off values for each gene correspond to the expression level of said gene which correlates with the highest specificity at said desired sensitivity in a ROC (receiver operating characteristics) curve calculated based on the expression levels of the PCA3, PSMA and PSGR genes determined in a patient population being at risk of suffering prostate cancer,

wherein a significant increase in the expression level of at least one of said genes in said sample with respect to said predetermined cut off value for said gene is indicative that the subject suffers from PCa with said desired sensitivity.

In another aspect, the invention relates to a method for assessing or monitoring the response to a therapy in a subject having prostate cancer (PCa) which comprises comparing

(i) determining the expression levels of the PCA3, PSMA and PSGR genes in a sample isolated from said subject obtained prior to the administration of said therapy,
(ii) determining the expression levels of the PCA3, PSMA and PSGR genes in a sample isolated from said subject obtained after the administration of said therapy,
(iii) comparing the expression levels of said PCA3, PSMA and PSGR genes obtained prior to and after the administration of said therapy with predetermined cut off values for each of said genes wherein said predetermined cut off values for each gene correspond to the expression level of said gene which correlates with the highest specificity at said desired sensitivity in a ROC (receiver operating characteristics) curves calculated based on the expression levels of the PCA3, PSMA and PSGR genes determined in a patient population being at risk of suffering prostate cancer,

wherein a significant decrease in expression levels of at least one of said genes in the subject sample after the administration of said therapy with respect to said predetermined cut off value for said gene is indicative that the therapy administered is efficacious

or

wherein a significant increase or a lack of change in the expression levels of at least one of said genes in the subject sample after the administration of said therapy with respect to said predetermined cut off value for said gene is indicative that the therapy administered is inefficacious.

In yet another aspect, the invention relates to a method for monitoring the progression of prostate cancer (PCa) in a subject, which comprises

(i) Determining the expression levels of the PCA3, PSMA and PSGR genes in a subject sample obtained at a first period of time and
(ii) Determining the expression levels of said genes PCA3, PSMA and PSGR genes in a sample of the same subject obtained at a second period of time, wherein said second period of time is later than said first period of time;
(iii) comparing the expression levels of said PCA3, PSMA and PSGR genes obtained at the first and at the second period of time with predetermined cut off values for each of said genes wherein said predetermined cut off values for each gene correspond to the expression level of said gene which correlates with the highest specificity at said desired sensitivity in a ROC (receiver operating characteristics) curves calculated based on the expression levels of the PCA3, PSMA and PSGR genes determined in a patient population being at risk of suffering prostate cancer,

wherein a significant decrease in expression levels of at least one of said genes in the subject sample at the second period of time with respect to said expression level at the first period of time is indicative of a positive progression of the subject

or

wherein a significant increase or a lack of change in expression levels of at least one of said genes in the subject sample at the second period of time with respect to said expression level at the first period of time is indicative of a negative progression of the subject.

In another aspect, the invention relates to a method for assessing if a subject has to be subjected to a prostate biopsy, said subject having a serum PSA range within 4-10 ng/mL and no previous biopsy, which comprises:

(i) determining the level of expression of genes PCA3, PSMA and PSGR in a sample isolated from said subject and
(ii) comparing the expression levels of said PCA3, PSMA and PSGR genes with predetermined cut off values for each of said genes wherein said predetermined cut off values for each gene correspond to the expression level of said gene which correlates with the highest specificity at said desired sensitivity in a ROC (receiver operating characteristics) curve calculated based on the expression levels of the PCA3, PSMA and PSGR genes determined in a patient population being at risk of suffering prostate cancer,

4

wherein a significant increase in the expression level of at least one of said genes in said sample with respect to said predetermined cut off value for said gene is indicative that the subject is candidate for prostate biopsy.

In another aspect, the invention relates to a kit comprising a first component and, optionally, a second component wherein the first component is a set of reagents consisting of:

    i) a reagent which allows determining the expression level of gene PCA3;
    ii) a reagent which allows determining the expression level of gene PSMA; and
    iii) a reagent which allows determining the expression level of gene PSGR; and

wherein the second component comprises consists of one or more reagents which allow the determination of the expression levels of one or more prostate housekeeping genes.

In an additional aspect, the invention relates to the use of a kit according to claim 16 for the diagnosis of PCa, for assessing or monitoring the response to therapy in a subject having PCa or for monitoring the progression of PCa in a subject.

## BRIEF DESCRIPTION OF THE DRAWINGS

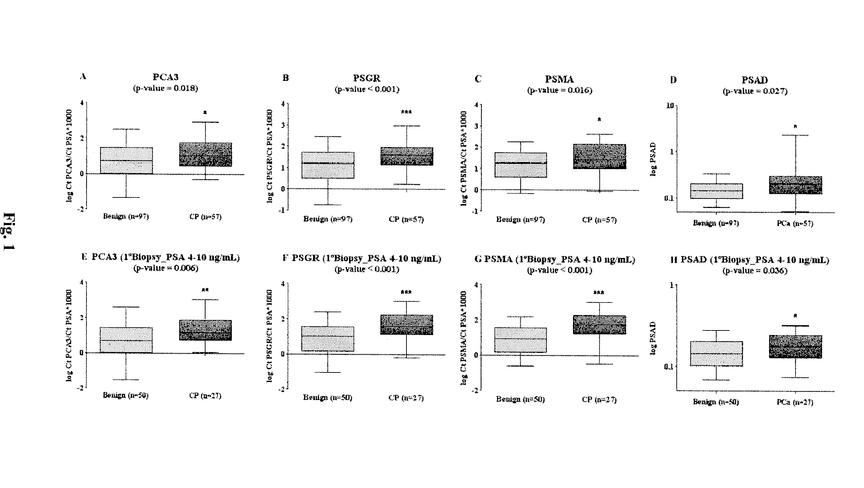

    **Figure 1.** Characterization of urine-based PCa biomarkers. Figure 1.1 shows the relative level of PCA3 (A), PSGR (B), PSMA (C) and PSAD (D) in men with PCa vs. benign and the relative level of PCA3 (E), PSGR (F), PSMA (G) and PSAD (H) in men with PCa vs. benign in the serum PSA 4-10 ng/mL and no previous biopsy (group of patients of special clinical interest).

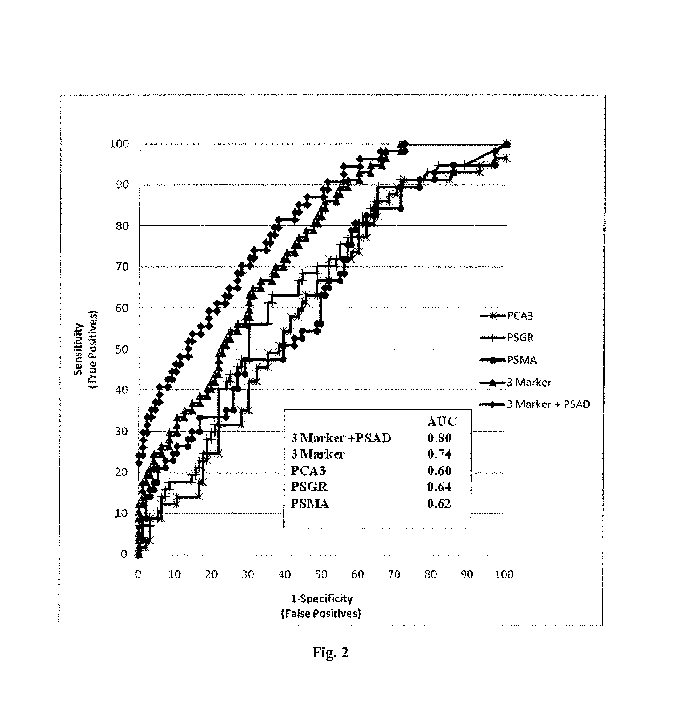

    **Figure 2.** Receiver-operating-characteristic (ROC) curves for the combined biomarker in men with prostate cancer detected and men with no cancer detected at biopsy. ROC curve for PCA3 (x), ROC curve for PSGR (•) and ROC curve for PSMA (V), and PSAD (x) Combined 3 Gene-ROC (♦). Combined 3 Gene+PSAD-ROC curve (□).

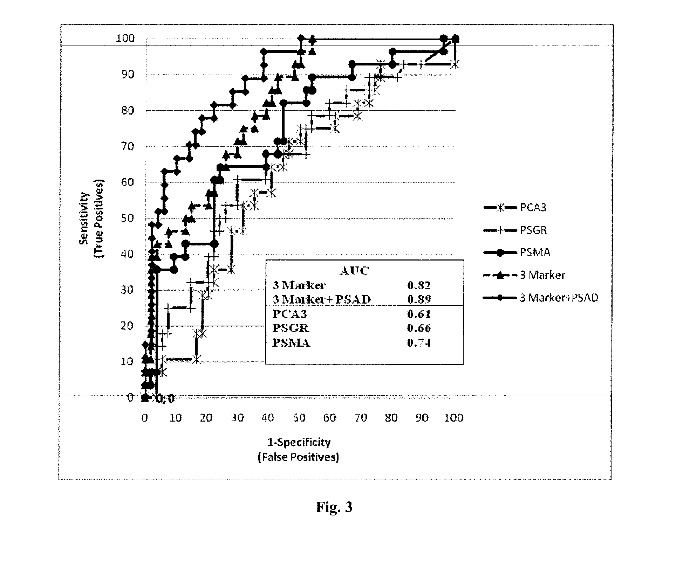

    **Figure 3.** Receiver-operating-characteristic (ROC) curves comparing individual markers and a combined biomarker (PCA3, PSGR, PSMA + PSAD) in men with PSA 4-10 ng/mL and no previous biopsy. ROC curve for PCA3 (x), ROC curve for PSGR (●) and ROC curve for PSMA (V), and PSAD (x) Combined 3 Gene-ROC (♦). Combined 3 Gene+PSAD-ROC curve (□).

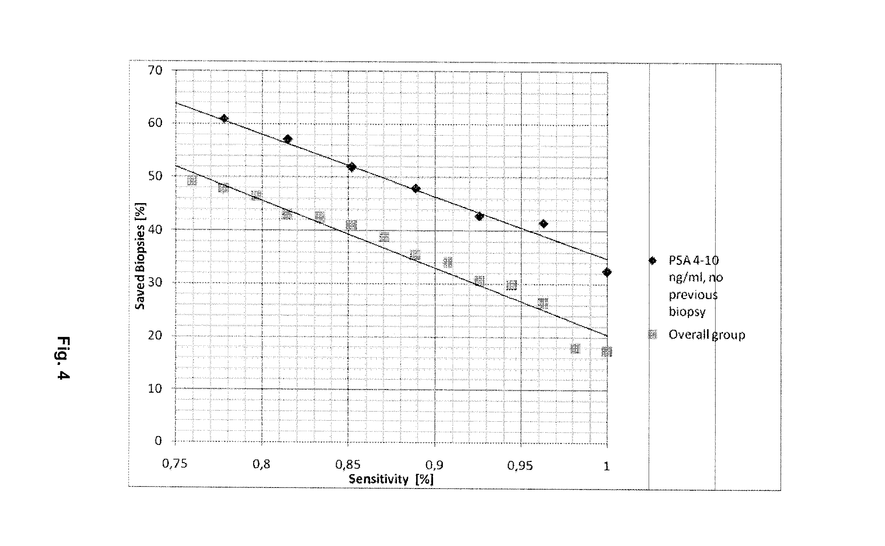

    **Figure 4.** Biopsies that could be saved comparing all patients from the study with a clinical risk group of special interest (PSA in the range 4-10 ng/mL and no previous biopsies). Biopsies saved(%) = (True negatives + False negatives) / All patients. Sensitivity = True positives / (True positives + False negatives).

## DETAILED DESCRIPTION OF THE INVENTION

Diagnostic method of the invention

The authors of the present invention have identified a combination of specific genes which are differentially expressed in tumor samples of patients diagnosed with PCa with respect to non-PCa reference samples which, when analysed in a combined ROC-analysis, allow obtaining a combined marker which allows detection of prostate cancer with an improved performance. For instance, as shown in figure 2 of the present invention, the combined ROC analysis allows detection of prostate cancer with a AUC of 0.80, which is improved compared to the AUC for individual markers alone (0.61 for PCA3; 0.64 for PSGR and 0.63 for PSMA).

Thus, in a first aspect, the invention relates to a method for the diagnosis of prostate cancer (PCa) with a desired sensitivity in a subject which comprises

    (i) determining the expression levels of the PCA3, PSMA and PSGR genes in a sample isolated from said subject,
    (ii) comparing the expression levels of said PCA3, PSMA and PSGR genes with predetermined cut off values for each of said genes wherein said predetermined cut off values for each gene correspond to the expression level of said gene which correlates with the highest specificity at said desired sensitivity in a ROC (receiver operating characteristics) curve calculated based on the expression levels of the PCA3, PSMA and PSGR genes determined in a patient population being at risk of suffering prostate cancer,

wherein a significant increase in the expression level of at least one of said genes in said sample with respect to said predetermined cut off value for said gene is indicative that the subject suffers from PCa with said desired sensitivity.

As it is used herein, the expression "method for the diagnosis" relates to a method that may essentially consist of the previously mentioned steps or may include additional steps. However, it must be understood that the method, in a preferred embodiment, is a method that is carried out *in vitro,* i.e., it is not carried out in the human or animal body. Diagnosing as used herein relates to evaluating the probability according to which a subject suffers from a disease. As it will be understood by persons skilled in the art, such evaluation, although it is preferred that it is, normally may not be correct for 100% of the subjects to be diagnosed. The term, however, requires that a statistically significant part of the subjects can be identified as suffering from the disease or having a predisposition for it. The person skilled in the art can determine if a part is statistically significant without further delay by using several well known statistic evaluation tools, for example, determination of confidence intervals, determination of the p value, Student's t-test, Mann-Whitney test, etc. The details are in Dowdy and Wearden, Statistics for Research, John Wiley & Sons, New York 1983. The preferred confidence intervals are of at least 50%, at least 60%, at least 70%, at least 80%, at least 90%, at least 95%, preferably of at least 99%. The p values are preferably 0.2, 0.1, 0.05, preferably 0.01. The method for the diagnosis of the invention allows to assess whether a subject is afflicted with PCa.

The term "prostate cancer" refers to any cancer of the prostate gland in which cells of the prostate mutate and begin to multiply out of control irrespective of the stage of the cancer. The extent to which prostate cancer has progressed in a patient is assessed taking into account clinical and histopathological information. The stage of cancer is classified based on tumour size (T), whether there is lymph node involvement (N), the presence of metastasis (M), and the tumour grading (G). A tumour classed as T1 is confined to the prostate gland and too small to be felt by digital rectal examination. T1 further includes T1a (fewer than 5% cancerous cells in tissue sample) and T1b (more than 5%) subdivisions. T1c indicates the patient has an elevated Prostate Specific Antigen (PSA; see definition later). If the tumour is large enough to be felt during a digital rectal examination, it is classified as T2. T2a means only one side of the prostate gland (left or right) is involved; T2b means both sides have a tumour(s). T2 is commonly termed "localized cancer". If the cancer is T3, it has spread to the connected tissue near the prostate (T3a) or the seminal vesicles (T3b). T4 indicates cancer spread to tissue next to the prostate, for example the bladder sphincter, rectum or pelvis wall. The prostate cancer may also spread into the regional lymph nodes of the pelvis and this is assessed as N1 stage of prostate cancer. These stages of T3, T4 and N1 are collectively termed "locally advanced" or regional cancer. If the cancer has spread to distant sites, such as the bone, it is said to be "metastasized" or at the M1 stage. Prostate cancer that has spread to distant lymph nodes is categorized as M1 a while that which has spread to bone is M1b and that which has spread to organs such as liver or brain is assessed as M1c. Left untreated, prostate cancer almost universally metastasizes to bone.

A "subject", as used herein, refers to a any animal classified as mammal and includes but is not limited to domestic and farm animals, primates and humans, for example, human beings, non-human primates, cows, horses, pigs, sheep, goats, dogs, cats, or rodents. Preferably, the subject is a male or female human being of any age or race.

In a preferred embodiment, the subject wherein the determination is carried out shows PSA levels which are higher than 4 ng/mL. In a still more preferred embodiment, the method of the invention is carried out in a subject showing PSA levels lower than 10 ng/mL and, more preferably, between 4 and 10 ng/mL. In another embodiment, the method of the invention is carried out in subjects who have not been subjected to prostate biopsy.

In a preferred embodiment, the subject wherein the method of the invention is applied refers to a patient suffering isolated high-grade prostate intraepithelial neoplasia.

The term "high-grade prostate intraepithelial neoplasia" or "HG-PIN", as used herein, refers to a condition associated with high risk of prostate cancer and also known as dysplasia, intraductal dysplasia, large acinar atypical hyperplasia, atypical primary hyperplasia, hyperplasia with malignant change, marked atypia, and duct-acinar dysplasia. HG-PIN may be characterized by a high nuclear/cytoplasmic ratio, hyperchromasia, coarsely granular chromatin, absence of nucleoli, isolated cells and cellular and nuclear pleomorphism. Methods used for diagnosis of PIN are known in the art and include, but are not limited to, needle biopsy and measurement of expression of ezrin, a cytoskeleton linker protein that is actively involved in regulating the growth and metastatic capacity of cancer cells {see, Pang et ai, Urology. 2004 Mar; 63(3):609-12, and U.S. Patent No. 6,054,320 provides kits for identification of PIN.

Thus, patients diagnosed with HG-PIN are particularly suitable candidates for the method of the invention as they are at high risk of developing prostate cancer as a result of malignant conversion of the HG-PIN.

The term "sensitivity", as used herein, refers to the probability that a diagnostic method of the invention gives a positive result when the sample is positive. Sensitivity is calculated as the number of true positive results divided by the sum of the true positives and false negatives. Sensitivity essentially is a measure of how well a method correctly identifies those with disease, i.e. prostate cancer. In a method of the invention, the cut off values can be selected such that the sensitivity of diagnosing an individual is at least about 70 %, and can be, for example, at least 75 %, at least 80 %, at least 85 %, at least 90 %, at least 95 %, at least 96 %, at least 97 %, at least 98 %, at least 99 % or at least 100% in at least 60 % of the patient population assayed, or in at least 65 %, 70 %, 75 % or 80 % of the patient population assayed.

In a first step, the diagnostic method of the invention comprises the determination of the expression levels of the PCA3, PSMA and PSGR genes in a sample isolated from the subject to be diagnosed.

The expression "expression levels of one or more genes" as used in accordance with the method of the invention relates to the degree of gene expression of one or more genes. Typically, the expression levels of a given gene can be determined by measuring the RNA expression level, wherein the term "RNA expression level" refers to a determined level of the converted DNA gene sequence information into transcribed RNA, the initial unspliced RNA transcript or the mature mRNA.

RNA expression can be monitored by measuring the levels of either the entire RNA of the gene or subsequences. Virtually any method for detecting the presence of and for quantifying the level of a gene can be used within the frame of the instant invention in order to detect and quantify the levels of mRNA encoded by the biomarker genes. By way of a non-limiting illustration, in a particular embodiment, for measuring the amount of a particular RNA in a sample, methods known to one of ordinary skill in the art can be used to extract and quantify transcribed RNA from a sample with respect to the biomarker genes (PSGR). Please, see WO2008/121132 and WO 98/24935 herein incorporated by reference for RNA analysis protocols. Briefly, RNA is extracted from a sample such as any tissue, body fluid, cell (e.g., circulating tumor cell), etc. For example, cells may be lysed and RNA eluted in a suitable solution in which to conduct a DNAse reaction. Subsequent to RNA extraction, first strand synthesis may be performed using a reverse transcriptase. Gene amplification, more specifically quantitative PCR assays, can then be conducted and the gene of interest calibrated against an internal marker such as, e.g., 18S rRNA, although any other endogenous marker can also be used, such as 28S-25S rRNA and 5S rRNA. Samples are measured in multiple replicates, for example, 3 replicates. In an embodiment of the invention, qPCR is performed using amplification, reporting agents and instruments such as those supplied commercially by Applied Biosystems. Given a defined efficiency of amplification of target transcripts, the point (e.g., cycle number) that signal from amplified target template is detectable may be directly related to the amount of specific message transcript in the measured sample. Similarly, other quantifiable signals such as fluorescence, enzyme activity, disintegrations per minute, absorbance, etc., when correlated to a known concentration of target templates (e.g., a reference standard curve) or normalized to a standard with limited variability can be used to quantify the number of target templates in an unknown sample.

Although not limited to amplification methods, quantitative gene expression techniques may use amplification of the target transcript. Alternatively, or in combination with amplification of the target transcript, quantification of the reporter signal for an internal marker generated by the exponential increase of amplified product may also be used. Amplification of the target template may be accomplished by isothermic gene amplification strategies or by gene amplification by thermal cycling such as PCR.

Specific RNAs are amplified using message specific primers or random primers. The specific primers can be synthesized from data obtained from public databases (e.g., Unigene, National Center for Biotechnology Information, National Library of Medicine, Bethesda, MD), including information from genomic and cDNA libraries obtained from humans and other animals. Primers are chosen to preferentially amplify from specific RNAs obtained from the test or indicator samples [see, for example, RT PCR, Chapter 15 in RNA Methodologies. A Laboratory Guide for Isolation and Characterization. 2nd edition, 1998, Robert E. Farrell, Jr., Ed., Academic Press; or Chapter 22 pp.143-151, RNA Isolation and Characterization Protocols. Methods in Molecular Biology, Volume 86, 1998, R. Rapley and D. L. Manning Eds., Human Press, or Chapter 14 Statistical refinement of primer design parameters; or Chapter 5, pp.55-72, PCR Applications: protocols for functional genomics, M.A.Innis, D.H. Gelfand and J.J. Sninsky, Eds., 1999, Academic Press]. Amplifications are carried out in either isothermic conditions or using a thermal cycler [for example, a ABI 9600 or 9700 or 7900 obtained from Applied Biosystems]. Amplified nucleic acids are detected using fluorescent-tagged detection oligonucleotide probes [see, for example, Taqman™ PCR Reagent Kit, Protocol, part number 402823, Revision A, 1996, Applied Biosystems] that are identified and synthesized from publicly known databases as described for the amplification primers.

For example, without limitation, amplified cDNA is detected and quantified using a suitable detection system, e.g., the ABI Prism® 7900 Sequence Detection System (Applied Biosystems). Amounts of specific RNAs contained in the test sample can be related to the relative quantity of fluorescence observed (see, for example, Advances in Quantitative PCR Technology: 5' Nuclease Assays, Y.S. lie and CJ. Petropolus, Current Opinion in Biotechnology, 1998, 9:43-48, or Rapid Thermal Cycling and PCR Kinetics, pp. 211-229, chapter 14 in PCR applications: protocols for functional genomics, M. A. Innis, D.H. GelfandandXJ. Sninsky, Eds., 1999; Academic Press).

In a particular embodiment, quantitative PCR (qPCR) is used to detect and quantify the levels of expression of the PSGR. PCA3 and PSMA genes. Conventional methods of quantifying the genes expression levels can be found, for example, in Sambrook et al., 2001 "Molecular cloning: to Laboratory Manual", 3rd ed., Cold Spring Harbor Laboratory Press, N.Y., Vol. 1-3.

The term "PCA3" refers to the gene known as prostate cancer antigen 3 (Accesion number in NCBI is AF103907 or NR_015342) corresponds to a non-protein coding gene located on chromosome 9 which encodes a prostate-specific non-coding RNA which is highly over expressed in more than 95% of primary PCa specimens and PCa metastasis.

The term "PSMA", as used herein, refers to the gene known as prostate specific membrane antigen), also known as FOLH1 (folate hydrolase (prostate-specific membrane antigen) 1), which encodes at least two transcript variants (identified in NCBI as NM_004476 and NM_001014986) which encode integral non-shed type 2 membrane proteins that are highly and specifically expressed on prostate epithelial cells and strongly up-regulated in PCa as well as in the neovasculature of other solid tumors [Elsasser-Beile, U. et al.. Curr Drug Targets 10:118-25 (2009)].

The term "PSGR", as used herein, refers to the prostate-specific G-protein coupled receptor), also known as ORS51E2 (olfactory receptor, family 51, subfamily E, member 2, NM_030774), which is a member of G-protein coupled OR family that is highly prostate tissue-specific and its tumor-associated overexpression.

For certainty, the polynucleotides useful to practice the invention include without being limited thereto mutants, homologs, subtypes, alleles and the like. It shall be understood that if is not required that the sequences of the present invention encode functional variants of the PCA3, PSMA and PSGR genes as long as the variant genes show a correlation between their expression levels and the presence of prostate cancer similar to that of the natural genes. The term variant, as used herein, refers to polynucleotides resulting from the deletion, insertion or substitution of one or more nucleotides from the PSGR polynucleotide. Variants of the PSGR include, without limitation, polynucleotides showing a degree of identify of at least 70%, advantageously of at least 75%, typically of at least 80%, preferably of at least 85%, more preferably of at least 90%, still more preferably of at least 95%, 97%, 98% or 99%, with respect to the PSGR mRNA. The degree of identity between two polynucleotide sequences can be determined by conventional methods, for example, by means of standard sequence alignment algorithms known in the state of the art, such as, for example, BLAST [Altschul S.F. et al. Basic local alignment search tool. J Mol Biol. 1990 Oct 5; 215(3):403-10].

In the present invention, the term "sample" or "biological sample" means biological material isolated from a subject and may include a single cell or multiple cells or fragments of cells or an aliquot of body fluid, taken from the subject, by any suitable means, e.g., venipuncture, excretion, ejaculation, massage, biopsy, needle aspirate, lavage sample, scraping, surgical incision or intervention or other means known in the art. The biological sample should contain any biological material suitable for detecting the desired biomarker; thus, said sample should, advantageously comprise cell material, e.g., circulating tumor cells, from the subject. Thus, the sample, which should provide a source of nucleic acid from prostate tissue or cells, can be isolated from any suitable tissue or biological fluid, such as, for example, prostate cells, prostate tissue, prostatic fluid, urine, ejaculate, semen, etc. In practice, since prostate cells can be detected in the urine of men with PCa, the use of fluid such as urine is recommended. In a particular embodiment, the samples used for the determination of the biomarkers according to the present invention are preferably urine samples obtained after a prostate massage (e.g., DRM) or after prostate biopsy (post-biopsy) or by spontaneous miction, preferably, urine samples obtained after a prostate massage (e.g., DRM) since it appears to be the best compromise between a minimal invasive technique accepted by a wider range of the male population and the possibility to obtain enough cells for a correct diagnosis. In another embodiment, the samples used for the determination of the biomarkers according to the present invention are samples obtained by expressed prostatic secretions or prostatectomy (useful as negative controls).

In a second step, the method of the invention comprises comparing the expression levels of said PCA3, PSMA and PSGR genes with predetermined cut off values for each of said genes wherein said predetermined cut off values for each gene correspond to the expression level of said gene which correlates with the highest specificity at said desired sensitivity in a ROC (receiver operating characteristics) curve calculated based on the expression levels of the PCA3, PSMA and PSGR genes determined in a patient population being at risk of suffering prostate cancer.

The term "cut off value", when referring to the expression levels PCA3, PSMA and PSGR genes, refer to a reference expression level indicative that a subject is likely to suffer prostate cancer with a given sensitivity if the expression levels of the patient are above said cut-off or reference leves. Typically, cut-off values are calculated using a Receiver Operating Characteristic curve (ROC curve).

In practice, Receiver Operating Characteristic curves (ROC curves), are typically calculated by plotting the value of a variable versus its relative frequency in "normal" (i.e. apparently healthy) and "disease" populations. For any particular marker or panel of markers (i.e., the expression levels of said PCA3, PSMA and PSGR genes), a distribution of marker levels for subjects with and without a disease will likely overlap. Under such conditions, a test does not absolutely distinguish normal from disease with 100 percent accuracy, and the area of overlap indicates where the test cannot distinguish normal from disease. A threshold or cut-off value is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art. See, e.g., Hartley et al. 1982. Radiology 143: 29-36. Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5 percent of a given measurement.

The horizontal axis of the ROC curve represents (1-specificity), which increases with the rate of false positives.

The vertical axis of the curve represents sensitivity, which increases with the rate of true positives. Thus, for a particular cut-off selected, the value of (1- specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In certain embodiments, particular thresholds for one or more markers in a panel are not relied upon to determine if a profile of marker levels obtained from a subject are indicative of a particular diagnosis/prognosis. Rather, the present invention may utilize an evaluation of a marker panel "profile" as a unitary whole. A particular "fingerprint" pattern of changes in such a panel of markers may, in effect, act as a specific diagnostic or prognostic indicator. As discussed herein, that pattern of changes may be obtained from a single sample, or from temporal changes in one or more members of the panel (or a panel response value). A panel herein refers to a set of markers.

As described herein after, a panel response value is preferably determined by plotting ROC curves for the sensitivity (i.e. true positives) of a particular panel of markers versus 1-(specificity) (i.e. false positives) for the panel at various cut-offs. In these methods, a profile of marker measurements from a subject is considered together to provide a global probability (expressed either as a numeric score or as a percentage risk) of a diagnosis or prognosis. In such embodiments, an increase in a certain subset of markers may be sufficient to indicate a particular diagnosis/prognosis in one patient, while an increase in a different subset of markers may be sufficient to indicate the same or a different diagnosis/prognosis in another patient. Weighting factors may also be applied to one or more markers in a panel, for example, when a marker is of particularly high utility in identifying a particular diagnosis/prognosis, it may be weighted so that at a given level it alone is sufficient to signal a positive result. Likewise, a weighting factor may provide that no given level of a particular marker is sufficient to signal a positive result, but only signals a result when another marker also contributes to the analysis.

For a marker measured on continuous scales, a ROC curve is a plot of true positive fraction versus false positive fraction, evaluated for all possible cut-off point values. For binary outcome, i.e., presence of prostate cancer or not, the ROC curve quantifies the discriminatory ability of a marker for separating cases from controls. The standard deviations of the area under the curve (AUC) and the differences between AUCs are computed with the U-statistic of DeLong et al. (Biometrics, 1988, 44:837-845) or the bootstrap re-sampling method.

Preferably, a ROC curve is determined for k markers by firstly using a k-component detection threshold for each biomarker followed by declaring the test as positive if at least one of the scores was above its detection threshold. Sensitivity and specificity values are tipically calculated over the range of k-component thresholds which allows the generation of a cloud of points. The optimal ROC curve points for the new marker can be obtained in the following way: for a fixed sensitivity value, the maximum value for specificity was selected among the range of specificities in the cloud of points that matched that sensitivity value.

In certain embodiments, markers and/or marker panels are selected to exhibit at least about 70 percent sensitivity, more preferably at least about 80 percent sensitivity, even more preferably at least about 85 percent sensitivity, still more preferably at least about 90 percent sensitivity, and most preferably at least about 95 percent sensitivity, combined with at least about 70 percent specificity, more preferably at least about 80 percent specificity, even more preferably at least about 85 percent specificity, still more preferably at least about 90 percent specificity, and most preferably at least about 95 percent specificity. In particularly preferred embodiments, both the sensitivity and specificity are at least about 75 percent, more preferably at least about 80 percent, even more preferably at least about 85 percent, still more preferably at least about 90 percent, and most preferably at least about 95 percent. The term "about" in this context refers to +/- 5 percent of a given measurement.

The skilled artisan will understand that associating a diagnostic or prognostic indicator, with a diagnosis or with a prognostic risk of a future clinical outcome is a statistical analysis. For example, a marker level of greater than X may signal that a patient is more likely to suffer from an adverse outcome than patients with a level less than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, e.g., Dowdy and Wearden, Statistics for Research, John Wiley and Sons, New York, 1983. Preferred confidence intervals of the invention are 90 percent, 95 percent, 97.5 percent, 98 percent, 99 percent, 99.5 percent, 99.9 percent and 99.99 percent, while preferred p values are 0.1, 0.05, 0.025, 0.02, 0.01, 0.005, 0.001, and 0.0001.

The term "patient population being at risk of suffering prostate cancer", as used herein, refers to a population of patients which have been diagnosed as showing risk of suffering prostate cancer based on one or more of the assays available for detecting prostate cancer. The population, for example, can comprise 3, 4, 5, 10, 15, 20, 30, 40, 50 or more subjects.

Patients being at risk of suffering prostate cancer include, without limitation, patients showing one or more of the following diagnostic traits:

- Positive for digital rectal examination (DRE), i.e. Presence of areas which are irregular, hard or lumpy detected by a procedure where the examiner inserts a gloved, lubricated finger into the rectum to check the size, shape, and texture of the prostate as described by Richie JP et al. (Urology, 1993, 42:365-74 and Carvalhal GF et al. (J. Urol., 1999, 161:835-9). Although the DRE only evaluates the back of the prostate, 85 percent of prostate cancers arise in this part of the prostate. Prostate cancer which can be felt on DRE is generally more advanced.
- Transrectal ultrasound, also known as prostate ultrasound, ultrasound scanning or sonography, involves exposing part of the body to high-frequency sound waves to produce pictures of the inside of the body. Because ultrasound images are captured in real-time, they can show the structure and movement of the body's internal organs, as well as blood flowing through blood vessels.

- Elevated PSA levels in blood: Although no standard exists as to the decision level for biopsies in patients showing increased PSA levels in blood, different values of PSA have been chose arbitrarily as decision level, in particular, levels above 4 ng/mL were chosen as decision level for biopsies in the clinical trial upon which the FDA in 1994 based adding prostate cancer detection in men age 50; 4 ng/mL was used as the biopsy decision level in the PLCO trial, 3 ng/mL was used in the ERSPC and ProtecT trials, and 2.5 ng/mL is used in the 2007 NCCN guideline. PSA levels can change for many reasons other than cancer. Two common causes of high PSA levels are enlargement of the prostate (benign prostatic hypertrophy (BPH)) and infection in the prostate (prostatitis).
- Increased levels in blood of the prostatic acid phosphatase (PAP).

In a preferred embodiment, the patients of the patient population being at risk of suffering prostate cancer are patients having PSA levels above 4 ng/mL and/or patients with a positive DRE. In a still more preferred embodiment, the patient population being at risk of suffering prostate cancer is formed by patients having PSA levels found within the so-called grey zone which corresponds to PSA levels in serum or blood associated with an undesirably large numbers of false positives and false negatives. Typically, the grey zone corresponds to PSA levels higher than 4 ng/mL and lower than 10 ng/mL.

In a more preferred embodiment, the subject wherein the method of the invention is carried out is a patient wherein no previous biopsy has been carried out.

Once the levels of the PCA3, PSMA and PSGR genes in a given patient have been compared with the expression levels for each respective gene determined as conferring the maximum specificity at the desired sensitivity using a multimarker ROC curve analysis, a patient is diagnosed as having prostate cancer with the desired sensitivity when the expression level of at least one of the above genes is above said predetermined expression level. The term "a significant increase in the expression level" of a gene in a sample of the subject under study, as used herein, refers to increases in the expression levels of with respect to the cut-off values by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more.

As used herein, the term "specificity" means the probability that a diagnostic method of the invention gives a negative result when the sample is not positive. Specificity is calculated as the number of true negative results divided by the sum of the true negatives and false positives. Specificity essentially is a measure of how well a method excludes those who do not have prostate cancer. In a method of the invention, the cut-off values for the expression of PCA3, PSMA and PSGR genes can be selected such that, when the sensitivity is at least about 70 percent, the specificity of diagnosing an individual is in the range of 70-100 percent, for example, at least 75 percent, 80 percent, 85 percent, 90 percent or 95 percent in at least 60 percent of the patient population assayed, or in at least 65 percent, 70 percent, 75 percent or 80 percent of the patient population assayed. As illustrated in the examples of the present invention, the method allows diagnosing the presence of prostate cancer with a sensitivity of 96% and a specificity was 40%, being the positive and negative predictive values were 48% and 95%, respectively (Figure 2).

The term "negative predictive value," as used herein, is synonymous with "NPV" and means the probability that an individual diagnosed as not having prostate cancer actually does not have the disease. Negative predictive value can be calculated as the number of true negatives divided by the sum of the true negatives and false negatives.

The term "positive predictive value," as used herein, is synonymous with "PPV" and means the probability that an individual diagnosed as having fibrosis actually has the condition. Positive predictive value can be calculated as the number of true positives divided by the sum of the true positives and false positives.

The method of the present invention allows the detection of prostate cancer with a desired sensitivity when at least one of the markers measured in the method of the invention are expressed above the cut-off level taken as reference without substantially reducing the specificity of the diagnostic method. This surprising effect is thought to result from the fact that altered expression of the different genes may vary from one patient to the other depending on the origin of the tumor, the tumor subtype, the degree of progression. In this situation, determination of a single gene or pairwise combinations of the genes would result in some cases not been properly diagnosed, thus decreasing sensitivity. The method according to the invention allows an increased sensitivity without affecting specificity by measuring the expression of all three genes.

As the skilled person will appreciate, the diagnosis can be positive if the expression levels of at least PCA3, of at least PSMA or of at least PSGR are above the cut-off levels. Moreover, a positive diagnostic is also found when the expression levels of two out of the three above genes are above the reference values for each gene such as, for instance, when PCA3 and PSMA levels are elevated, when PCA3 and PSGR are elevated or when PCA3 and PSGR are elevated. Additionally, a positive diagnosis results when the expression levels of PCA3, PSMA and PSGR are simultaneously elevated.

The method for the diagnosis of PCa provided by the instant invention is a non-invasive method since it can be performed by using urine samples containing prostate cells from the subject under analysis, and reliable due to its high sensitivity and specificity (a specificity of 40% is obtained at a sensitivity of 96%); in addition, the positive and negative predictive values are 48% and 95%, respectively. Therefore, taken together, these results can be used to develop a highly specific test of PCa which could be easily integrated in the routine urologist workflow and could reduce significantly the high number of unnecessary biopsies due to the low specificity of the PSA test particularly in group of patients of special clinical interest (patients with serum PSA 4-10 ng/mL and no previous biopsy) as it will be discussed below.

In a preferred embodiment, the expression levels of the PCA3, PSMA and PSGR genes are normalized using the expression levels of a housekeeping gene to take into consideration that an increased expression levels of the genes in the sample may not be the result of the malignant proliferation of the prostate cells but rather of a non-malignant prostate growth as it occurs for instance in the benign hyperplasia of the prostate. Moreover, the method can be carried out in a sample containing cells from tissues other than prostate which express these markers. In particular, if the method is carried out in urine sediments, besides cancer cells, it is possible to find cells from the urothelium, kidney, bladder or blood. Thus, in a preferred embodiment, the expression levels of the different genes forming the marker panel are normalized to the amount of a housekeeping gene.

The term "normalization", as used herein, means conversion of the quantitative numerical values into numerical values which can be compared with gene expression amounts obtained by other gene expression analyses. Typically, normalization is carred out by using the gene expression levels of a housekeeping gene being steadily expressed is used as an index for normalization of gene expression amounts.

The term "housekeeping gene", as used herein, refers to a gene which is generally ubiquitously expressed in all tissues. These genes encode proteins that provide the basic, essential functions that all cells need to survive. Housekeeping genes are usually expressed at the same level in all cells and tissues, but with some variances, especially during cell growth and organism development. Commonly used housekeeping genes include, without limitation, GAPDH (glyceraldehyde-3-phosphate dehydrogenase), beta-actin gene, the tubulin gene, genes coding for the 18S or 28S rRNA subunits of the ribosome. An exemplary listing of housekeeping genes can be found, for example, in Trends in Genetics, 19, 362-365 (2003).

In a preferred embodiment, the housekeeping gene is a "prostate housekeeping gene". As used herein, the term "prostate housekeeping gene" refers to a gene which is expressed at steady levels in prostate cells irrespective of whether the cells are normal prostate cells or prostate adenocarcinoma cells. This gene allows the detection of the number of prostate derived cells in a sample

Thus, in a preferred embodiment, the first method of the invention comprises the use of expression levels of the PCA3, PSMA and PSGR genes after normalization to the expression level of a prostate housekeeping gene in the same sample wherein the PCA3, PSMA and PSGR are determined.

Suitable prostate housekeeping genes for use in the present invention include, without limitation, PSA (Sokoll et al., 1997, Urol. Clin. North Am. 24:253-259), DD3 (Cancer Res., 1999, 59:5975-9), HPG-1, PSM, NKX3, six-transmembrane epithelial antigen of prostate (STEAP), Trp-p8 (Tsvaler et al.), prostatic acid phosphatase, creatine kinase, thymosin b-15, HPC1 basic prostate gene, the prostate-specific glandular kallikrein protein hK2 encoded by the hKLK2 gen, the prostate-specific antigen protein, hK3 (PSA) encoded by the hKLK3 gene, the SIM2 gene (WO09135019A), the ERG gene (WO09135019A), TMPRSS2, PSM or prostate-specific membrane antigen (Fair et al., 1997, Prostate 32:140-148), PSCA or prostate stem cell antigen (Reiter et al., 1998. Proc. Natl. Acad. Sci. USA 95:1735-1740), TMPRSS2 (Lin et al., 1999. Cancer Res. 59:4180-4184), PDEF (Oettgen et al., 2000, J. Biol. Chem. 275:1216-1225), PSG-1 or prostate-specific genre-1 (Herness, 2003, Cancer Res. 63:329-336), PCA3 (Bussemakers et al., 1999. ,Cancer Res. 59:5975-5979], WO98/045420, WO01/0235WO2004/070056, WO2005/003387), PCGEM1 (Srikantan et al., 2000. Proc. Natl. Acad. Sci. USA 97:12216-12221) and the genes P704P, P712P, and P775P (Stolk et al., 2004. Prostate 60: 214-226).

In a preferred embodiment, the prostate housekeeping gene is the PSA gene. The term PSA, as used herein, refers to a gene located on chromosome 19 which encodes a glycoprotein with serine protease activity expressed under androgen control by glandular epithelial cells of the prostate and secreted into seminal plasma to liquefy it. PSA protein is normally confined to the prostate but in the case of prostatic disease such as cancer or BPH (benign prostate hyperplasia), PSA leaks into the blood where it is present in different forms, including one that is and one that is not bound to protein complexes (El-Shirbiny, 1994, Adv. Clin. Chem. 31:99). The measurement of total PSA serum concentrations is one of the most frequently used and FDA approved biochemical tests in the screening and management of prostate cancer patients. Studies to date have suggested that screening with PSA, in conjunction with digital rectal exams and transrectal ultrasound, increases the detection of early prostate cancers often while still localized to the gland itself (Brawer et al., 1992, J. Urol. 147:841). Serum PSA is also useful for monitoring of patients after therapy, especially after surgical prostatectomy. However, total PSA measurements also identify a large number of patients with abnormally elevated levels who are subsequently found to have no prostate cancer. Recently, the concept of measuring the percentage free/total PSA ratio was shown to increase the specificity of prostate cancer screening in men with PSA between 4 and 10 ng/mL (Letran et al., 1998, J. Urol. 160:426).

The authors of the present invention have also observed that the diagnostic value of the marker panel can be further increased by incorporating a fourth maker, namely, the PSA density or PSAD.

Thus, as shown in the examples of the present invention, a marker panel comprising the expression levels of PCA3, PSMA and PSGR as well as PSAD allows the diagnosis of prostate cancer with an AUC of 0.89, whereas the use of the three expression levels without PSAD results in an AUC of 0.82. The sensitivity for the combined marker model 96% and the specificity was 40%. The positive and negative predictive values were 48% and 95%, respectively (see Figure 2).

The incorporation of the four marker to the diagnostic method requires (i) the determination of the PSAD value in the patient under study, (ii) the comparison of the PSAD value with a cut-off value for said parameter wherein said cut-off value corresponds to the cut-off value which provides the highest specificity at the desired sensitivity using a ROC curve calculated using the combined four marker panel.

Thus, in a preferred embodiment, the diagnostic method of the invention further comprises in step (i) the determination of PSAD in the patient, in step (ii) the comparison of the PSAD level in the patient with a predetermined cut-off value for PSAD wherein said predetermined cut off value corresponds to a PSAD value which correlates with the highest specificity at said desired sensitivity in a ROC (receiver operating characteristics) curve calculated based on the expression levels of the PCA3, PSMA, PSGR genes and PSAD determined in a patient population being at risk of suffering prostate cancer and, finally, taking a decision on whether a patient shows prostate cancer wherein increased expression level of at least one of said genes in said sample with respect to said predetermined cut off value for said gene or increased PSAD values with respect to said predetermined cut off value is indicative that the subject suffers from PCa with said desired sensitivity.

The term "PSA density" or PSAD , as used herein, refers to the PSA value obtained by dividing the PSA value from the sample of bodily fluid by the prostate volume.

For example: PSA density (PSD) = PSA value / prostate volume

The PSAD (PSA/prostate volume) value can be determined by measuring the concentration of PSA in a sample (e.g., serum, blood, etc., preferably, serum) and the prostate volume (ecography) following the formula

$$PSA\ density = PSA\ volume/prostate\ volume$$

The concentration of PSA in a sample (e.g., serum, blood, etc.) can be determined by any method for determining the concentration of PSA in a sample including conventional methods well known by the skilled person in the art as described above (e.g., immunological methods, etc.).

The prostate volume can be measured by any method for measuring the volume of prostate including conventional methods well known by the skilled person in the art including, without limitation, the so-called TRUS examination using the formula HxWxLx0.52. The prostate volume is typically determined based on ultrasound measurements, including, but not limited to, transrectal ultrasound measurements (TRUS) taken in the greatest dimension. In yet another particular embodiment, the TRUS measurements are calculated by the ellipsoid volume method of HxWxLx0.52. The volume may be calculated by taking measurements of the entire prostate gland, or alternatively, the volume of the transition zone (TZ)/periurethral benign prostate glandular lobe (adenoma) may also be obtained and the values used to determine PSA density.

"Transrectal ultrasound" or "TRUS" is a 5- to 15-minute outpatient procedure that uses sound waves to create a video image of the prostate gland. The procedure involves the placement of a small, lubricated probe into the rectum which releases sound waves, which create echoes as they enter the prostate. Prostate tumors often create echoes that are different from normal prostate tissue. The echoes that bounce back are sent to a computer that translates the pattern of echoes into a picture of the prostate. TRUS is used to estimate the weight of the prostate gland, helping doctors get a better idea of PSA density, which helps distinguish benign prostatic hyperplasia (BPH) from prostate cancer.

"Ellipsoid volume method for measuring prostate volume" is a method for assessing the volume of the prostate and is an important and integral part of the TRUS procedure. Several formulas have been used, but the most common one is the ellipsoid formula, which requires measurement of 3 prostate dimensions. Dimensions are first determined in the axial plane by measuring the transverse and anteroposterior dimension at the estimated point of widest transverse dimension. The longitudinal dimension is measured in the sagittal plane just off the midline because the bladder neck often obscures the cephalad extent of the gland. The ellipsoid volume formula is then applied, as follows: Volume=height.times.width.times.length.times.0.52

Alternatively, the prostate volume can be determined non-invasively by using a combination of PSA, cPSA (PSA to $\alpha$1-antichymotrypsin), free PSA, B-PSA (benign PSA); PRO-PSA (precursor isoform of free PSA, which is associated with prostate cancer and which is comprised of native proPSA as well as truncated proPSA forms, [-2]pPSA and [-4]pPSA) and/or human kallikrein (HK2) measurements.

The method of the invention allows the identification of patients suffering prostate cancer wherein at least one of the above markers is significantly increased with respect to the cut-off value. As the skilled person will appreciate, the diagnosis can be positive if the expression levels of at least PCA3, of at least PSMA or of at least PSGR or of at least PSAD are above the cut-off levels. Moreover, a positive diagnostic is also found when the expression levels of two out of the three markers are above the reference values for each gene such as, for instance, when PCA3 levels and PSGR levels are elevated, when PCA3 levels and PSMA levels are elevated, when PCA3 levels and PSAD levels are elevated, when PSGR and PSMA levels are elevated, when PSGR levels and PSAD are elevated or when PSMA levels and PSAD are elevated. Additionally, a positive diagnosis results when the expression levels of PCA3, PSMA and PSGR are simultaneously elevated, when the expression levels of PCA3, PSMA and the PSAD value are simultaneously elevated, when the expression levels of PCA3 and PSGR and the PSAD value are simultaneously elevated and when the expression levels of PSGR and PSMA and the PSAD are simultaneously elevated.

Assessing or monitoring the response to PCa therapy

The teachings of the invention can be used for monitoring and determining the efficacy of the therapy administered to a subject having PCa.

Thus, in another aspect, the invention relates to a method for assessing or monitoring the response to a therapy in a subject having prostate cancer (PCa) which comprises comparing

(i) determining the expression levels of the PCA3, PSMA and PSGR genes in a sample isolated from said subject obtained prior to the administration of said therapy,
(ii) determining the expression levels of the PCA3, PSMA and PSGR genes in a sample isolated from said subject obtained after the administration of said therapy,
(iii) comparing the expression levels of said PCA3, PSMA and PSGR genes obtained prior to and after the administration of said therapy with predetermined cut off values for each of said genes wherein said predetermined cut off values for each gene correspond to the expression level of said gene which correlates with the highest specificity at said desired sensitivity in a ROC (receiver operating characteristics) curves calculated based on the expression levels of the PCA3, PSMA and PSGR genes determined in a patient population being at risk of suffering prostate cancer,

wherein a significant decrease in expression levels of at least one of said genes in the subject sample after the administration of said therapy with respect to said predetermined cut off value for said gene is indicative that the therapy administered is efficacious

Alternatively, in another aspect, the invention relates to a method for assessing or monitoring the response to a therapy in a subject having prostate cancer (PCa) which comprises comparing

(i) determining the expression levels of the PCA3, PSMA and PSGR genes in a sample isolated from said subject obtained prior to the administration of said therapy,
(ii) determining the expression levels of the PCA3, PSMA and PSGR genes in a sample isolated from said subject obtained after the administration of said therapy,
(iii) comparing the expression levels of said PCA3, PSMA and PSGR genes obtained prior to and after the administration of said therapy with predetermined cut off values for each of said genes wherein said predetermined cut off values for each gene correspond to the expression level of said gene which correlates with the highest specificity at said desired sensitivity in a ROC (receiver operating characteristics) curves calculated based on the expression levels of the PCA3, PSMA and PSGR genes determined in a patient population being at risk of suffering prostate cancer,

wherein a significant increase or a lack of change in expression levels of at least one of said genes in the subject sample after the administration of said therapy with respect to said predetermined cut off value for said gene is indicative that the therapy administered is inefficacious.

As used in the present invention, the expression "assessing or monitoring the response to therapy" relates to the possibility of determining the response of a subject having PCa to the therapy administered to said subject. In PCa therapy, a variety of treatments can be used in an attempt to eliminate or contain the cancer. Treatment for PCa may involve active surveillance, hormonal therapy, surgery (e.g., radical prostatectomy, transurethral resection of the prostate (TURP), cryosurgery), radiation therapy including brachytherapy (prostate brachytherapy) and external beam radiation, High Intensity Focused Ultrasound (HIFU), chemotherapy, or some combination. Which option is best depends on the stage of the disease, the Gleason score, and the PSA level. Other important factors are the man's age, his general health, and his feelings about potential treatments and their possible side effects. Because all treatments can have significant side effects, such as erectile dysfunction and urinary incontinence, treatment discussions often focus on balancing the goals of therapy with the risks of lifestyle alterations.

Briefly, depending on the above mentioned factors (e.g., age of the subject, as well as the size, location and cancer phase), (i) surgery, consisting of the surgical extirpation of the tumor (ii) cytotoxic/cytostatic treatments, such as chemotherapy, which uses medicinal products against cancer to destroy the cancerous cells upon making the medicinal products circulate through the body through the blood vessels; radiotherapy, which uses high energy radiations to kill the cancer cells, and antitumor agents; and/or (iii) immunotherapy, wherein the administered compound stimulates, enhances or strengthens the natural function of the immune system against cancer to recognize and eliminate the cancerous cells from the body, can be used. Thus, the method of the invention allows determining the response of the subject having PCa to any treatment, particularly, to any cytotoxic and/or cytostatic treatment and, more specifically, to a treatment by means of chemotherapy, radiotherapy, antitumor agents or combinations thereof.

Suitable chemotherapy agents include but are not limited to alkylating agents [e.g., Cisplatin, Carboplatin, Oxaliplatin, BBR3464, Chlorambucil, Chlormethine, Cyclophosphamides, Ifosmade, Melphalan, Carmustine, Fotemustine, Lomustine, Streptozocin, Busulfan, Dacarbazine, Mechlorethamine, Procarbazine, Temozolomide, ThioTPA, Uramustine, etc.]; anti-metabolites [e.g., purine (azathioprine, mercaptopurine), pyrimidine (Capecitabine, Cytarabine, Fluorouracil, Gemcitabine), folic acid (Methotrexate, Pemetrexed, Raltitrexed), etc.]; vinca alkaloids [e.g., Vincristine, Vinblastine, Vinorelbine, Vindesine, etc.]; a taxane [e.g., paclitaxel, docetaxel, BMS-247550, etc.]; an anthracycline [e.g., Daunorubicin, Doxorubicin, Epirubicin, Idarubicin, Mitoxantrone, Valrubicin, Bleomycin, Hydroxyurea, Mitomycin, etc.]; a topoisomerase inhibitor [e.g., Topotecan, Irinotecan Etoposide, Teniposide, etc.]; a monoclonal antibody [e.g., Alemtuzumab, Bevacizumab, Cetuximab, Gemtuzumab, Panitumumab, Rituximab, Trastuzumab, etc.]); a photosensitizer [e.g., Aminolevulinic acid, Methyl aminolevulinate, Porfimer sodium, Verteporfin, etc.]; a tyrosine kinase inhibitor [e.g., Gleevec $^{(TM)}$] ; an epidermal growth factor receptor inhibitor [e.g., Iressa $^{(TM)}$ erlotinib (Tarceva $^{(TM)}$), gefitinib, etc.]; an FPTase inhibitor [e.g., FTIs (Rl 15777, SCH66336, L- 778,123), etc.]; a KDR inhibitor [e.g., SU6668, PTK787, etc.]; a proteosome inhibitor [e.g., PS341, etc.]; a TS/DNA synthesis inhibitor [e.g., ZD9331, Raltirexed (ZD 1694, Tomudex), ZD9331, 5-FU, etc.]; an S-adenosyl-methionine decarboxylase inhibitor [e.g., SAM468A, etc.]; a DNA methylating agent [e.g., TMZ, etc.]; a DNA binding agent [e.g., PZA, etc.]; an agent which binds and inactivates $O^6$-alkylguanine AGT [e.g., BG]; a c-ra/-1 antisense oligo-deoxynucleotide [e.g., ISIS-5132 (CGP- 69846A)]; tumor immunotherapy; a steroidal and/or non-steroidal antiinflammatory agent [e.g., corticosteroids, COX-2 inhibitors]; or other agents such as Alitretinoin, Altretamine, Amsacrine, Anagrelide, Arsenic trioxide, Asparaginase, Bexarotene, Bortezomib, Celecoxib, Dasatinib, Denileukin Diftitox, Estramustine, Hydroxycarbamide, Imatinib, Pentostatin, Masoprocol, Mitotane, Pegaspargase, and Tretinoin. Suitable chemotherapy agents are described with more detail in the literature, such as in The Merck Index in CD-ROM, 13th edition.

According to this inventive aspect, the expression level of genes PCA3, PSMA and PSGR determined in the sample from a subject having PCa obtained at a first period of time (first subject sample) and the expression level of genes PCA3, PSMA and PSGR determined in the sample from a subject having PCa obtained at a second period of time (second subject sample) are compared allowing the efficacy of the response to therapy in said subject having PCa to be assessed or monitorized. The second subject sample can be taken from the same subject having PCa from which the first measure is derived, at a second period of time, i.e., at any time after the first period of time, e.g., one day, one week, one month, two months, three months, 1 year, 2 years, or more after the first subject sample. In a particular embodiment, the first subject sample is taken prior to the subject receiving treatment, e.g. chemotherapy, radiation therapy, or surgery, and the second subject sample is taken after treatment. In another particular embodiment, the first subject sample is taken after the subject has started/received treatment, e.g. chemotherapy, radiation therapy, or surgery, and the second subject sample is taken later, at different time periods during a course of treatment which efficacy is to be assessed or monitorized. These methods allow for the evaluation of a particular treatment for a selected subject previously diagnosed with PCa. Consequently, if the therapy is not efficacious for treating PCa in said subject, then said therapy should be changed and a new therapy should be designed to treat PCa in said subject. The course of the new treatment can be easily followed according to this method.

As mentioned previously concerning the diagnostic method of the invention, the level of expression of the biomarker genes (PCA3, PSMA and PSGR) can be determined by any suitable means known in the art, such as, for example, qPCR. The measurement is obtained under conditions that are substantially repeatable.

Once the expression levels of the biomarker genes in the subject samples at different period of time (first and second subject samples) have been determined, it is necessary to identify if there is a significant decrease in the expression of each one of said genes in the second subject sample in comparison with the expression levels of said gene biomarkers in the first subject sample. A decrease in the expression of a gene in the second subject sample under study is considered a "significant decrease" with respect to the expression level of said biomarkers genes in said first subject sample when the expression level in the second subject sample decreases with respect to the expression level thereof in the first subject sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

Similarly, an increase in the expression of a gene in the second subject sample under study is considered a "significant increase" with respect to the expression level of said biomarkers genes in said first subject sample when the expression level in the second subject sample increases with respect to the expression level thereof in the first subject sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more.

Similarly, a lack of change in the expression of a gene in the second subject sample under study with respect to the expression level of said biomarkers genes in said first subject sample when the expression level in the second subject sample indicates that the expression levels are substantially constant between the two measurements. By way of example, constant expression levels indicate that the first measurement is not more than 105%, not more than 104%, not more than 103%, not more than 102%, not more than 101%, not less than 99%, not less than 98%, not less than 97%, not less than 97%, not less than 96% or not less than 95%.

Thus, a significant decrease in the expression level of at least one of said biomarker genes (PCA3, PSMA and PSGR) in the second subject sample with respect to the expression level of each of said biomarker genes in the first subj ect sample is indicative that the therapy administered to the subject having PCa is efficacious. On the contrary, if no significant decrease (lack of alteration or significant increase) in the level of expression of at least of said biomarker genes in the second subject sample with respect to the expression level of each of said biomarker genes in the first subject sample is achieved, or even, if a significant increase in the level of expression of at least one of said biomarker genes in the second subject sample with respect to the expression level of at least one of said biomarker genes in the first subject sample is achieved, then the therapy administered to said subject is not efficacious for treating PCa in said subject; consequently, said therapy should be changed and a new therapy should be designed to treat PCa in said subject. The course of the new treatment can be easily followed according to this method.

Moreover, the method for assessing or monitoring the response to PCa therapy according to the present invention can be further improved by combining the expression levels of the different genes with the PSAD value so that a therapy is considered as being efficacious when the expression level of at least one of the above genes or at least the PSAD value is lower than the cut-off value or a therapy is considered as being inefficacious when the expression level of at least one of the above genes or at least the PSAD value is higher than the cut-off value.

Thus, in a preferred embodiment, the method for monitoring the response to a therapy according to the present invention is carried out by performing steps (i) and (ii) as defined above but wherein steps (i) and (ii) further comprise the determination of PSAD in the patient, wherein step (iii) further comprises comparing the PSAD level in the patient with a predetermined cut-off value for PSAD wherein said predetermined cut off value corresponds to a PSAD value which correlates with the highest specificity at said desired sensitivity in a ROC (receiver operating characteristics) curves calculated based on the expression levels of the PCA3, PSMA, PSGR genes and PSAD determined in a patient population being at risk of suffering prostate cancer. Finally, the method allows obtaining a conclusion as to whether a therapy is being efficacious if a decreased expression level of at least one of said genes in said sample with respect to said predetermined cut off value for said gene or decreased PSAD values with respect to said predetermined cut off value is detected or as to whether a therapy is being inefficacious if an increase or a lack of change in the expression levels of at least one of said genes in said sample with respect to said predetermined cut off value for said gene or an increase or lack of change in PSAD values with respect to said predetermined cut off value is detected.

Monitoring the progression of PCa in a subject

The teachings of the invention can also be used for monitoring the progression of PCa in a subject. Thus, in another aspect, the invention relates to a method for monitoring the progression of prostate cancer (PCa) in a subject, which comprises

a) Determining the expression levels of the PCA3, PSMA and PSGR genes in a subject sample obtained at a first period of time and

b) Determining the expression levels of said genes PCA3, PSMA and PSGR genes in a sample of the same subject obtained at a second period of time, wherein said second period of time is later than said first period of time;

c) comparing the expression levels of said PCA3, PSMA and PSGR genes obtained at the first and at the second period of time with predetermined cut off values for each of said genes wherein said predetermined cut off values for each gene correspond to the expression level of said gene which correlates with the highest specificity at said desired sensitivity in a ROC (receiver operating characteristics) curves calculated based on the expression levels of the PCA3, PSMA and PSGR genes determined in a patient population being at risk of suffering prostate cancer,

wherein a significant decrease in expression levels of at least one of said genes in the subject sample at the second period of time with respect to said expression level at the first period of time is indicative that the prostate cancer is not progressing in the subject.

Alternatively, the method for monitoring the progression of prostate cancer (PCa) in a subject comprises

a) Determining the expression levels of the PCA3, PSMA and PSGR genes in a subject sample obtained at a first period of time and

b) Determining the expression levels of said genes PCA3, PSMA and PSGR genes in a sample of the same subject obtained at a second period of time, wherein said second period of time is later than said first period of time;

c) comparing the expression levels of said PCA3, PSMA and PSGR genes obtained at the first and at the second period of time with predetermined cut off values for each of said genes wherein said predetermined cut off values for each gene correspond to the expression level of said gene which correlates with the highest specificity at said desired sensitivity in a ROC (receiver operating characteristics) curves calculated based on the expression levels of the PCA3, PSMA and PSGR genes determined in a patient population being at risk of suffering prostate cancer,

wherein a significant increase or a lack of change in the expression levels of at least one of said genes in the subject sample at the second period of time with respect to said expression level at the first period of time is indicative of a progression of prostate cancer.

As used in the present invention, the expression "monitoring the progression of PCa", which is equivalent to "determining the prognosis", relates to the determination of one or several parameters indicating the progression of the disease in a patient diagnosed with PCa. Parameters suitable for determining the evolution of a subject diagnosed with PCa are selected from the group of tumor response, risk of relapse, disease-free survival and/or overall survival of the subject. As used herein, the expression "risk of relapse" is understood as the probability of a subject developing PCa and/or a secondary metastasis again after a disease-free period; "disease-free survival" is understood as the time period after the treatment in which the cancer is not found; and "overall survival of the subject" is understood as the percentage of subjects who survive, from the time of the diagnosis or treatment, after a defined time period. As used herein, disease progression means that the

According to this inventive aspect, the expression level of genes PCA3, PSMA and PSGR determined in the sample from a subject having PCa obtained at a first period of time (first subject sample) and the expression level of genes PCA3, PSMA and PSGR determined in the sample from a subject having PCa obtained at a second period of time (second subject sample) are each compared to cut-off values for each of the genes wherein said cut-off values are determined as defined above. The second subject sample can be taken from the same subject having PCa from which the first measure is derived, at a second period of time, i.e., at any time after the first period of time, e.g., one day, one week, one month, two months, three months, 1 year, 2 years, or more after the first subject sample. In a particular embodiment, the first subject sample is taken prior to the subject receiving treatment, e.g. chemotherapy, radiation therapy, or surgery, and the second subject sample is taken after treatment. In another particular embodiment, the first subject sample is taken after the subject has started/received treatment, e.g. chemotherapy, radiation therapy, or surgery, and the second subject sample is taken later, at different time periods during a course of treatment. These methods allow for the evaluation of the progression of the PCa in a selected subject previously diagnosed as suffering from PCa. Consequently, if the PCa has a bad prognosis, a further therapy should be designed to treat PCa in said subject. The progression of the PCa after said new treatment can be easily followed according to the teachings of this invention.

As mentioned previously concerning the diagnostic method of the invention, the level of expression of the biomarker genes (PCA3, PSMA and PSGR) can be determined by any suitable means known in the art, such as, for example, qPCR. The measurement is obtained under conditions that are substantially repeatable.

Once the expression levels of the biomarker genes in the subject samples, at different periods of time (first and second subject samples) have been determined, it is necessary to identify if there is a significant increase in the expression of at least one of said genes in the second subject sample in comparison with the expression levels of said gene biomarkers in the first subject sample. Alternatively, if desired, one may analyze if there is a significant decrease or a lack of change in the expression of at least one of said genes in the second subject sample in comparison with the expression levels of said gene biomarkers in the first subj ect sample. The terms "significant increase", "significant decrease" and "lack of change" applied to the expression levels of biomarkers genes have been previously defined.

Thus, a significant increase in the expression level of at least one of said biomarker genes (PCA3, PSMA and PSGR) in the second subject sample with respect to the expression level of each of said biomarker genes in the first subject sample is indicative that PCa in the subject under study is in progression (i.e., it has a bad prognosis); thus, the therapy administered to the subject under study should be changed and a new therapy should be designed to treat PCa in said subject. The progression of the PCa in the subject can be easily followed according to this method.

On the contrary, if no significant increase in the level of expression of at least one of said biomarker genes in the second subject sample with respect to the expression level of each of said biomarker genes in the first subject sample is achieved, or even, if a significant decrease in the level of expression of at least one said biomarker genes in the second subject sample with respect to the expression level of each of said biomarker genes in the first subject sample is achieved, then PCa in the subject under study is not in progression (i.e., it does not have a bad prognosis).

Moreover, the method for monitoring the progression of PCa according to the present invention can be further improved by combining the expression levels of the different genes with the PSAD value so that the disease is considered as not progressing when the expression level of at least one of the above genes or at least the PSAD value is lower than the cut-off value. Thus, in another aspect, the method for monitoring the response to a therapy according to the present invention is carried out by performing steps (i) and (ii) as defined above but wherein steps (i) and (ii) further comprise the determination of PSAD in the patient, wherein step (iii) further comprises comparing the PSAD level in the patient with a predetermined cut-off value for PSAD wherein said predetermined cut off value corresponds to a PSAD value which correlates with the highest specificity at said desired sensitivity in a ROC (receiver operating characteristics) curves calculated based on the expression levels of the PCA3, PSMA, PSGR genes and PSAD determined in a patient population being at risk of suffering prostate cancer. Finally, the method allows obtaining a conclusion as to whether PCa is progressing if an increased expression level or lack of change of at least one of said genes in said sample with respect to said predetermined cut off value for said gene or increased PSAD values or lack of change with respect to said predetermined cut off value is detected.

Selecting patients for biopsy

As mentioned above, the teachings of the invention can be used for selecting patients for biopsy. In fact, many biopsies are carried out which are unnecessary due to the low specificity of the PSA test. This is particularly the case in those patient having serum PSA levels in the range between 4.0-10.0 ng/mL, which has been described as a "gray zone", PSAD was significantly more accurate than total PSA [Ohori M, et al. Urology 46:666-71 (1995)].

As the authors of the present invention have shown, the application of the marker panel of the present invention allows a decision on whether the patient suffers prostate cancer with increased sensitivity and specificity, resulting in that the number of unnecessary biopsies could be reduced significantly. As shown for instance in the examples of the present invention, the number of biopsies that could be saved using the 3-component biomarker provided by the instant invention, in combination with PSAD, in the special clinical interest group (serum PSA range within 4-10 ng/mL and no previous biopsy), calculated as "saved biopsies = true negatives + false negatives", would be:

- with a sensitivity of 100%, 32.5% of the biopsies could be saved, and
- with a sensitivity of 96%, 41.6% of the biopsies could be saved,

what corresponds to about 126,750 to 162,240 saved biopsies/year throughout Europe.

Thus, in another aspect, the invention relates to a method for assessing whether a subject has to be subjected to a prostate biopsy, said subject having a serum PSA range within 4-10 ng/mL which comprises:

(i) determining the level of expression of genes PCA3, PSMA and PSGR in a sample isolated from said subject and
(ii) comparing the expression levels of said PCA3, PSMA and PSGR genes with predetermined cut off values for each of said genes wherein said predetermined cut off values for each gene correspond to the expression level of said gene which correlates with the highest specificity at said desired sensitivity in a ROC (receiver operating characteristics) curve calculated based on the expression levels of the PCA3, PSMA and PSGR genes determined in a patient population being at risk of suffering prostate cancer, wherein a significant increase in the expression level of at least one of said genes in said sample with respect to said predetermined cut off value for said gene is indicative that the subject is candidate for prostate biopsy.

The method for assessing whether a subject has to be subjected to a prostate can be improved by the determination of additional markers. In particular, the additional marker is PSAD (defined and calculated as explained above in the context of the diagnostic method of the invention).

The patient to be analysed according to the present method is preferably a patient which has not been subjected to a previous prostate biopsy. In another preferred embodiment, the patient which is analysed according to the present invention is a patient which has been subhected to at least one previous biopsy, at least two previous biopsies, at least three previous biopsies or more.

Thus, in a preferred aspect, the invention relates to a method selecting patients for biopsy according to the invention

wherein step (i) further comprises the determination of PSAD in the patient, wherein step (ii) further comprises comparing the PSAD level in the patient with a predetermined cut-off value for PSAD wherein said predetermined cut off value corresponds to a PSAD value which correlates with the highest specificity at said desired sensitivity in a ROC (receiver operating characteristics) curve calculated based on the expression levels of the PCA3, PSMA, PSGR genes and PSAD determined in a patient population being at risk of suffering prostate cancer and

wherein increased expression level of at least one of said genes in said sample with respect to said predetermined cut off value for said gene or increased PSAD values with respect to said predetermined cut off value is indicative that the the subject is candidate for prostate biopsy.

Kits of the invention

In another aspect, the invention relates to a kit comprising a first component and, optionally, a second component wherein the first component is a set of reagents consisting of:

(i) a reagent which allows determining the expression level of gene PCA3;
(ii) a reagent which allows determining the expression level of gene PSMA; and
(iii) a reagent which allows determining the expression level of gene PSGR; and

wherein the second component consists of one or more reagents which allow the determination of the expression levels of one or more prostate housekeeping genes.

The kit of the invention can be used for the diagnosis of PCa, i.e., for assessing whether a patient is afflicted with PCa, or for assessing or monitoring the response to therapy in a subject having PCa, i.e., for assessing the effect of a treatment in a subject diagnosed of PCa, or for monitoring the progression of PCa in a subject, i.e., for determining the prognosis of a subject diagnosed with PCa.

In the context of the present invention, "kit" is understood as a product containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet adDRMsses that provide said instructions.

A "reagent which allows determining the expression level of a gene" means a compound or set of compounds that allows determining the expression level of a gene, e.g., the determination of the level of the corresponding mRNA, etc., e.g., primers for the synthesis of the corresponding cDNA by means of RT, primers for the amplification of DNA, probes capable of specifically hybridizing with the mRNAs (or the corresponding cDNAs) encoded by said genes, Taqman probes, etc.

In a particular embodiment, the kit of the invention is designed to be used in a qPCR assay. Further, on a preferred embodiment, the first component of the kit of the invention consists of a specific Taqman probe for each one of genes PCA3, PSMA and PSGR. Gene expression assays used in qPCR analysis can be designed by a person skilled in the art or are commercially available from, for example, Applied Biosystems under codes Hs01371938-ml (PCA3), Hs00379515-ml (PSMA) and Hs00951952-ml (PSGR) (Example 1).

In another aspect, the invention relates to the use of a kit of the invention for the diagnosis of PCa, for assessing or monitoring the response to therapy in a subject having PCa, for monitoring the progression of PCa in a subject or for deciding whether a patient has to be subjected to prostate biopsy.

In a preferred embodiment, the use of the kit of the invention is carried out in a subject having serum PSA levels of 4-10 ng/mL.

The invention is detailed below by means of the following examples which are merely illustrative and by no means limiting for the scope of the invention.

**EXAMPLE 1**

**Identification of a three-gene panel for the early detection of PCa in urine**

**1. Materials and Methods**

1.1 Patients and urine collection

This study was approved by the institutional review board of the Vall d'Hebron Hospital. All urine samples were obtained from the Department of Urology of the Vall d'Hebron Hospital (Barcelona - Spain) and were taken from 198 men subjected to prostate biopsy, immediately after prostate massage (PM). Indications for biopsy were abnormal digital rectal examination (DRE) and/or serum PSA higher than 4 ng/mL. Written informed consent was obtained from all patients.
**PM methodology:** Systematically applying severe digital pressure to the prostate from the base to the apex and from the lateral to the median line of each lobe.
**Biopsy methodology and PCa detection rate:** The biopsies were performed using an end-fire ultrasound transducer (Falcon 2101, B-K Medical Inc) and an automatic 18 gauge needle (Bard Inc,). The minimal number of cores which were taken out in every procedure was 10, and between 1 and 8 additional cores were taken out according to the Vienna nomogram [Remzi et al., J. Urol. 2005; 174:1256-60; discussion 1260-1; author reply 1261].
For the total subject group studied, 154 of 198 specimens yielded sufficient RNA for analysis, corresponding to an informative specimen rate of 78% (91% patients with PCa and 28% benign). The PCa detection rate by a prostate biopsy was 37% (57/154). In a subgroup of 77 patients of special clinical interest to decide if a biopsy should be performed or not, PSA range within 4-10 ng/mL and no previous biopsy, the PCa detection rate was 36% (28/77) (Table 1).

**Table 1**

| Clinical and Pathological Information of the Informative Patients | | |
|---|---|---|
| | All patients | Patients PSA 4-10 ng/mL and no previous biopsy |
| No of patients | 154 | 77 |
| Age (yr) | 65.5 (44 - 85) | 64.8 (44-85) |
| PSA level (range) | 10.9 (2.5-189) | 6.6 (4.0-9.7) |
| Prostate Volume (mL) | 51.1 (162.0-10.0) | 46.2 (16.0-120.0) |
| PSA density (PSAD) | 1.61 (0.29-43.7) | 0.167 (0.06-0.34) |
| Ratio free PSA / total PSA | 0.17 (0.00-0.80) | 0.18 (0.01-0.80) |
| Prostate cancer | 57 (37%) | 28 (36%) |

1.2 Sample preparation

Urine samples (about 50 mL first catch) were collected in urine collection cups, kept on ice, transported to the laboratory and processed within 30 min. The urine samples were centrifugated at 2,500 xg for 10 minutes at 4°C, and then the pellets were washed twice with cold phosphate buffered saline (PBS) 1X. Finally, the pellets were stored with 1:5 RNA Later (Ambion) at -80°C until RNA extraction.

1.2.1 RNA isolation and preamplification

Urine RNA was extracted with the QIAamp® Viral RNA Mini Kit (Qiagen). Single-stranded cDNA synthesis was carried out using the SuperScript III Reverse Transcriptase (Invitrogen) and stored at -80°C until preamplification with the TaqMan Preamp Master Mix Kit (Applied Biosystems).

1.2.2 Quantitative PCR analysis

After previous studies, 3 putative PCa urinary biomarkers (PCA3, PSMA and PSGR) were selected by the inventors. Quantitative PCR (qPCR) was used to analyze said 3 putative PCa urinary biomarkers and the control transcript PSA, by using the TaqMan® Gene Expression Assay (Applied Biosystems). Reactions were carried out in triplicate on an ABI Prism 7900 qPCR apparatus, and only those results with an standard deviation (STDEV) lower than (<) 0.38 value were accepted. Threshold levels were set into the exponential phase of the qPCR. The data analysis was done using the ABI Prism 7900 SDS Software V2.3 (Applied Biosystems), with the same baseline and threshold set for each plate to generate threshold cycle (Ct) values for all the genes in each sample, in order to exclude the possibility that said markers might also be expressed in non-cancer cells normally found in urine sediments, such as cells from the urothelium, kidney, bladder or blood, the content in the clinical specimens was normalized to the amount of prostate derived RNA. Since only a relatively small number of prostate cells are to be found in urine, a cDNA preamplification step before the qPCR was performed. Inventors then established a qPCR cut-off of ct (PSA) lower than (<) 35 prior to preamplification, to determine whether a specimen was informative, i.e., the amount of RNA present was sufficient to yield an accurate result. For each marker a score was calculated as log (ct(marker)/ct(PSA) x 1000. A cut-off value for each marker was determined, and a specimen was counted positive when the marker was over the cut-off value.

1.3 <u>Statistical analysis</u>

The characterization of candidate biomarkers was accomplished by comparing their mean values between PCa and negative biopsy individuals. A t-test with Welch correction was used when the distribution of the data was Normal, and a Mann-Whitney test was used in other cases. Normality was verified with the Shapiro-Wilk normality test. All tests were carried out on log-transformed data, which were applied to stabilize the variances. Multivariate analysis was used to study the association between the candidate markers and the disease state. Receiver-Operating-Characteristic (ROC) curves and the Area Under the Curve (AUC) were used to evaluate the performance of each marker score as a measure to discriminate patients in the PCa group from others. ROC curves were calculated individually for each marker and for combinations of markers. In order to combine more than one marker ($k$), the following procedure was followed: first, a k-component detection threshold was used, one for each biomarker; then, the new marker was declared positive if at least one of the scores was above its detection threshold. Sensitivity and specificity values were calculated over the range of k-component thresholds. This generated a cloud of points. Optimal ROC curve points for the new marker were obtained in the following way: for a fixed sensitivity value, the maximum value for specificity was selected among the range of specificities in the cloud of points that matched that sensitivity value (Baker, S. G., J Natl Cancer Inst 2003, 95, 511-515). As the computational complexity increases exponentially when $k$ grows, only 3 markers were joined at the same time. The addition of a new variable to a $k$-multiplexed marker was carried out using the $k$-component cut-off values that had led to the optimal ROC curve points. These $k$-component thresholds were combined with the values of the new variable to generate ($k$+1)-component detection thresholds, which were used to obtain a new optimal ROC curve. Statistical analyses were performed using the PASW V17.0 and the free statistical language R and the ROC-package from the Bioconductor project (http://www.bioconductor.org).

**2. Results**

PCA3, PSGR, PSMA and additional clinical parameters were tested, in urine sediments obtained post-PM from 154 patients, to see whether they could discriminate patients with PCa from patients with negative needle biopsies. PCA3 (p=0.018), PSGR (p<0.001), PSMA (p=0.016) and PSAD (p=0.027) were statistically significant (Figure1.1 (A-D) box-and-whisker plots), while PSA (p=0.55), free PSA [fPSA] (p=0.60) and prostate volume [PST VOL] (p=0.053) did not show statistical significance (Figure 1.2 (A-C)).

After statistical analysis inventors chose PCA3, PSGR, PSMA, and PSAD for ROC-curve analysis to visualize the diagnostic efficacy and to summarize the data of the gene-based qPCR assay of the urine samples. The following AUC values were obtained: PCA3 (0.60), PSGR (0.64), PSMA (0.62) and PSAD (0.61) (Figure 2). The significance level P (Area=0.5) were p=0.043, 0.002, 0.012 and 0.051, respectively.

To determine if the combination of various biomarkers could improve performance over single biomarkers, a combined ROC analysis was performed. Therefore, inventors combined the 3 genes (PCA3, PSRG and PSMA) and PSAD (PSA/PST VOL) to a new marker in the following way: inventors used a 4-component marker detection threshold, one for each biomarker. The combined marker was declared positive if at least one marker was above its detection threshold. To draw the ROC curve inventors calculated sensitivity and specificity values over a range of 4-component thresholds. Thus, inventors were able to fix a value for sensitivity. The maximum value for specificity was selected from among the range of specificities that inventors had obtained matching the sensitivity value. The AUC for the combined marker model was 0.80 (0.74 without PSAD). At a sensitivity of 96% the specificity was 40%. The positive and negative predictive values were 48% and 95%, respectively (Figure 2). Table 2 contains the numerical values of said combination [(PCA3, PSRG and PSMA) and PSAD].

**Table 2**

| Optimal cut-off value of (PCA, PSRG and PSMA) and PSAD combination | | | | | |
|---|---|---|---|---|---|
| **Cut-off Value** | | | | | |
| **PCA3** | **PSGR** | **PSMA** | **PSA density** | **Sensitivity** | **Specificity** |
| 1.5 | 26.9 | 141.3 | 0.19 | 1.00 | 0.28 |
| 7.2 | 26.9 | 46.8 | 0.21 | 0.98 | 0.34 |
| 7.2 | 66.1 | 46.8 | 0.21 | 0.96 | 0.40 |
| 7.2 | 66.1 | 141.3 | 0.21 | 0.94 | 0.44 |
| 9772.4 | 33.9 | 44.7 | 0.21 | 0.91 | 0.49 |
| 9772.4 | 24.5 | 46.8 | 0.27 | 0.89 | 0.50 |
| 9772.4 | 38.0 | 128.8 | 0.21 | 0.87 | 0.54 |
| 9772.4 | 38.0 | 128.8 | 0.21 | 0.85 | 0.57 |
| 398.1 | 51.3 | 128.8 | 0.21 | 0.83 | 0.58 |
| 9772.4 | 33.9 | 141.3 | 0.27 | 0.81 | 0.62 |
| 9772.4 | 33.9 | 182.0 | 0.27 | 0.80 | 0.63 |
| 9772.4 | 38.0 | 128.8 | 0.27 | 0.78 | 0.64 |
| 398.1 | 177.8 | 128.8 | 0.21 | 0.76 | 0.66 |
| 398.1 | 51.3 | 128.8 | 0.27 | 0.74 | 0.69 |
| 3715.4 | 147.9 | 182.0 | 0.21 | 0.72 | 0.70 |
| 3715.4 | 147.9 | 182.0 | 0.21 | 0.70 | 0.72 |
| 3715.4 | 79.4 | 141.3 | 0.27 | 0.69 | 0.73 |
| 9772.4 | 51.3 | 141.3 | 0.41 | 0.65 | 0.76 |
| 398.1 | 177.8 | 128.8 | 0.27 | 0.63 | 0.77 |
| 398.1 | 147.9 | 182.0 | 0.27 | 0.61 | 0.79 |
| 3715.4 | 147.9 | 182.0 | 0.27 | 0.59 | 0.81 |
| 398.1 | 147.9 | 182.0 | 0.30 | 0.56 | 0.83 |
| 3715.4 | 147.9 | 182.0 | 0.30 | 0.54 | 0.86 |

Then inventors analyzed urine sediments obtained post-PM from 77 patients with PSA 4-10 ng/mL and no previous biopsy. This subgroup is of special clinical interest to decide if a biopsy should be performed or not.

All markers were tested by univariate analysis, if they could discriminate patients with PCa from patients with negative needle biopsies with the following results: PCA3 (p=0.006), PSGR (p<0.001), PSMA (p<0.001) and PSAD (p=0.036) were statistically significant (Figure 1), while PSA (p=0.42), fPSA (p=0.08) and PST VOL (p=0.021) did not show statistical significance (not shown). The AUC was: PCA3 (0.61), PSGR (0.66), and PSMA (0.74). The AUC for the combined marker model was 0.89 (0.82 without PSAD). At a sensitivity of 96% the specificity was 62%. The positive and negative predictive values were 57% and 97%, respectively (Figure 3). Table 3 contains the numerical values of said combination [(PCA3, PSRG and PSMA) and PSAD].

**Table 3**

| Optimal cut-off value of (PCA, PSRG and PSMA) and PSAD combination in a subgroup of patients of special clinical (serum PSA 4-10 ng/mL and no previous biopsy) | | | | | |
|---|---|---|---|---|---|
| **Cut-off Value** | | | | | |
| **PCA3** | **PSGR** | **PSMA** | **PSA density [ng/mL/mL]** | **Sensitivity** | **Specificity** |
| 13.5 | 162.2 | 39.8 | 0.27 | 1.00 | 0.50 |
| 3715.4 | 30.2 | 44.7 | 0.27 | 0.96 | 0.62 |
| 3715.4 | 26.9 | 128.8 | 0.27 | 0.89 | 0.68 |
| 3715.4 | 30.2 | 128.8 | 0.27 | 0.85 | 0.72 |
| 3715.4 | 162.2 | 128.8 | 0.21 | 0.81 | 0.78 |
| 3715.4 | 239.9 | 128.8 | 0.21 | 0.78 | 0.82 |
| 3715.4 | 239.9 | 128.8 | 0.21 | 0.74 | 0.84 |

(continued)

| Optimal cut-off value of (PCA, PSRG and PSMA) and PSAD combination in a subgroup of patients of special clinical (serum PSA 4-10 ng/mL and no previous biopsy) | | | | | |
|---|---|---|---|---|---|
| **Cut-off Value** | | | | | |
| **PCA3** | **PSGR** | **PSMA** | **PSA density [ng/mL/mL]** | **Sensitivity** | **Specificity** |
| 3715.4 | 239.9 | 128.8 | 0.23 | 0.70 | 0.86 |
| 3715.4 | 162.2 | 128.8 | 0.27 | 0.67 | 0.90 |
| 3715.4 | 239.9 | 128.8 | 0.27 | 0.63 | 0.94 |
| 3715.4 | 239.9 | 128.8 | 0.30 | 0.52 | 0.96 |
| 3715.4 | 239.9 | 128.8 | 0.31 | 0.48 | 0.98 |
| 3715.4 | 1122.0 | 295.1 | 0.30 | 0.15 | 1.00 |

Finally, inventors calculated the number of biopsies that could be saved using the 4 combined marker model in the overall group and in the clinical risk group of special interest, as "saved biopsies = true negatives + false negatives". With a sensitivity of 100%, 33% of the biopsies could be saved, and with a sensitivity of 96%, 42% of the biopsies could be saved (Figure 4).

## 3. Discussion

The use of proximal fluids, such as urine samples, after a prostate massage could be the best compromise between a minimally invasive technique and the possibility of obtaining enough cells for a correct diagnosis. The prostate acini communicating directly into the urethra allows mRNA to be detected from sloughed off prostate epithelial cells in urine voids. The hypothesis for this work was that a qPCR assay on sediments from voided urine samples obtained after PM might be capable of detecting a few malignant cells in a background of predominantly non-malignant cells and that a single marker may not necessarily reflect the multifactorial nature of PCa. For this reason, for a single marker in the range of sensitivities >95%, the specificity will drop dramatically. For example, for PCA3 at a sensitivity of 96%, a specificity of only 14% was found [Marks LS et al., Rev. Urol. 2008; 10:175-81]. In this context, PCa screening would benefit from a confirmatory test with a higher specificity., but the same sensitivity, and which could be used in conjunction with PSA testing to determine which patients should undergo biopsy. Consequently, inventors have developed a multiplexed qPCR-based test for PCa in urinary sediments after PM. Because the first portion of voided urine sample contains the highest concentration of prostatic and urethral secretions [Iwakiri J, et al. J Urol 149:783-6 (1993)], voided urine sample collection post-PM was selected. In addition, this type of sample is a more readily accepted specimen for men to provide, rather than ejaculate or expressed prostatic secretions.

The incorporation of additional clinical parameters can further improve diagnostic accuracy. One of this parameters could be the PSAD. In the serum PSA range 4.0-10.0 ng/mL, which was described as a "gray zone", PSAD was significantly more accurate than total PSA (Ohori M, et al. 1995, Urology 46:666-71). Therefore inventors included PSAD as a criterion for determining whether or not to perform prostate biopsy.

A prostate-specific mRNA, that is highly over-expressed in PCa cells, would be an ideal target. Because those genes may also be expressed in non-cancer cells normally found in urine sediments, such as cells from the urothelium, kidney, bladder or blood, its content in clinical specimens must be normalized to the amount of prostate derived RNA. This is achieved by using the ratio of gene/PSA mRNA concentrations as the diagnostic indicator. In contrast to the protein concentration in blood, PSA mRNA expression has been shown to be relatively constant in normal prostate cells, and only a weak down-regulation of PSA expression in PCa cells has been reported [Meng FJ, et al., Cancer Epidemiol Biomarkers Prev 11:305-9 (2002)]. The genes used in this study showed a high up-regulation in prostate tumors, compared to non-neoplastic prostate tissue. Therefore a down-regulation of PSA in prostate tumors would have caused the tumor-gene/PSA ratio to increase. As PSA is not expressed in other cells present in urine, such as transitional epithelium, blood or kidney, it can be used as a prostate gland housekeeping gene. To decide whether a specimen was informative, i.e., the amount of RNA present was sufficient to yield an accurate result, inventors used the PSA mRNA yield, as it is only present in prostate cells and would not be influenced by the varied content of other cell types present in the urine.

Although urine-based testing for PCA3 expression has already been documented in large screening programs, there are only two studies based on a diagnostic profile that take into account the heterogeneity of cancer development. A combination of various biomarkers should clearly improve performance over single biomarkers. To achieve this, inventors used a novel, multiplex panel of urine transcripts, generated by the combination of the 3 best markers (PCA3, PSGR and PSMA) and PSAD to a new marker in a combined ROC analysis. In the analyzed study groups, the AUC for the combined marker model (0.80) was notably improved, compared with the AUC for individual markers alone: PCA3 (0.60), PSGR (0.64) and PSMA (0.62). For example, a PCA3 score of 7.23, combined with a score of 65.51 for PSGR, 46.61 for PSMA and 0.21 PSAD, corresponded to the point on the ROC curve with high diagnostic accuracy: sensitivity and specificity (96% and 40%, respectively) (Figure 2). Analyzing only the patients with a serum PSA 4-10 ng/mL and no previous biopsy the combined marker model the specificity improved to 62% with 96% sensitivity and AUC (0.89) (Figure 3).

As inventors and previous studies used different methodologies to detect PCA3 transcripts in patient urine and many of them do not use the same prevalence of PCa (aprox 35%) in their study groups, directly comparing AUCs would have been inappropriate; however, inventors showed that PCA3 shows improved AUC when compared with the results from the above-mentioned studies or with serum PSA. These findings are consistent with previous reports [van Gils MP, et al. (2007) cited *supra;* Marks LS, et al. (2007) cited *supra;* Groskopf J, et al. (2006) cited *supra;* Hessels D, et al. (2003) cited *supra*]. Importantly, inventors have demonstrated that a combined model significantly improves predictive ability compared to PCA3 alone (Table 5 vs Table 6). The principle that underlies the combined biomarker approach is consistent with tests offered for the identification of high recurrence risk in breast cancer patients [Paik S, et al.; N Engl J Med 351:2817-26 (2004) y van de Vijver MJ, et al., N. Engl. J. Med. 347:1999-2009 (2002)]. In summary, inventors have shown that a multiplexed qPCR assay on urine sediments from patients presenting for prostate biopsy outperforms serum PSA or PCA3 alone.

The results of the combined 3-marker model + PSAD provided by the instant invention provided overall increased sensitivity without decreasing the specificity. Translated to the clinics, inventors achieved the same high sensitivity as the PSA-test alone, but increased the specificity considerable. Using this method at a sensitivity of 100%, 33% and with a sensitivity of 96%, 42% of the biopsies could be saved. This would correspond to a approximated yearly number of 126,750 to 162,240 saved biopsies throughout Europe.

As a possible limitation of this study and other studies the definition of patients negative for PCa used is based on recent negative biopsies. However this definition can be problematic because of 12% to 32% of patients with a negative prostate biopsy will actually be diagnosed with PCa at a later date [Cervera Deval J, et al. Actas Urol. Esp. 28: 666-71 (2004) and Raber M, et al., Arch. Ital. Urol. Androl. 72:197-9 (2000)]. Consequently, many men with negative biopsy findings undergo repeated biopsies to rule out PCa, thus, there will be a significant number of individuals with a positive test who are positive although the biopsy was negative. An accurate controlled approach will constitute validation of the biomarker profile in a prospective study or using retrospective studies on age matched patients with a 5-year follow up [Ouyang B, et al.. J. Urol., 181:2508-13; discussion 2513-4 (2009)]. Nevertheless, the data provided by the instant invention support the applicability of the 4-marker non-invasive assay method provided by the instant invention toward an accurate diagnostic test for PCa.

**Claims**

1.  A method for the diagnosis of prostate cancer (PCa) with a desired sensitivity in a subject which comprises

    (i) determining the expression levels of the PCA3, PSMA and PSGR genes in a sample isolated from said subject,
    (ii) comparing the expression levels of said PCA3, PSMA and PSGR genes with predetermined cut off values for each of said genes wherein said predetermined cut off values for each gene correspond to the expression level of said gene which correlates with the highest specificity at said desired sensitivity in a ROC (receiver operating characteristics) curve calculated based on the expression levels of the PCA3, PSMA and PSGR genes determined in a patient population being at risk of suffering prostate cancer,

    wherein a significant increase in the expression level of at least one of said genes in said sample with respect to said predetermined cut off value for said gene is indicative that the subject suffers from PCa with said desired sensitivity.

2.  A method according to claim 1
    wherein step (i) further comprises the determination of PSAD in the patient, wherein step (ii) further comprises comparing the PSAD level in the patient with a predetermined cut-off value for PSAD wherein said predetermined cut off value corresponds to a PSAD value which correlates with the highest specificity at said desired sensitivity in a ROC (receiver operating characteristics) curve calculated based on the expression levels of the PCA3, PSMA, PSGR genes and PSAD determined in a patient population being at risk of suffering prostate cancer and wherein increased expression level of at least one of said genes in said sample with respect to said predetermined cut off value for said gene or increased PSAD values with respect to said predetermined cut off value is indicative that the subject suffers from PCa with said desired sensitivity.

3. A method for assessing or monitoring the response to a therapy in a subject having prostate cancer (PCa) which comprises comparing

(i) determining the expression levels of the PCA3, PSMA and PSGR genes in a sample isolated from said subject obtained prior to the administration of said therapy,
(ii) determining the expression levels of the PCA3, PSMA and PSGR genes in a sample isolated from said subject obtained after the administration of said therapy,
(iii) comparing the expression levels of said PCA3, PSMA and PSGR genes obtained prior to and after the administration of said therapy with predetermined cut off values for each of said genes wherein said predetermined cut off values for each gene correspond to the expression level of said gene which correlates with the highest specificity at said desired sensitivity in a ROC (receiver operating characteristics) curves calculated based on the expression levels of the PCA3, PSMA and PSGR genes determined in a patient population being at risk of suffering prostate cancer,

wherein a significant decrease in expression levels of at least one of said genes in the subject sample after the administration of said therapy with respect to said predetermined cut off value for said gene is indicative that the therapy administered is efficacious
or
wherein a significant increase or a lack of change in the expression levels of at least one of said genes in the subject sample after the administration of said therapy with respect to said predetermined cut off value for said gene is indicative that the therapy administered is inefficacious.

4. A method according to claim 3
wherein steps (i) and (ii) further comprise the determination of PSAD in the patient and
wherein step (iii) further comprises comparing the PSAD level in the patient with a predetermined cut-off value for PSAD wherein said predetermined cut off value corresponds to a PSAD value which correlates with the highest specificity at said desired sensitivity in a ROC (receiver operating characteristics) curves calculated based on the expression levels of the PCA3, PSMA, PSGR genes and PSAD determined in a patient population being at risk of suffering prostate cancer, wherein significant decrease in the expression level of at least one of said genes in said sample with respect to said predetermined cut off value for said gene or decrease in PSAD values with respect to said predetermined cut off value is indicative that the therapy administered is efficacious
wherein a 3significant increase in expression levels of at least one of said genes in the subject sample after the administration of said therapy with respect to said predetermined cut off value for said gene or a significant increase in PSAD values with respect to said predetermined cut off value is indicative that the therapy administered is inefficacious.

5. A method for monitoring the progression of prostate cancer (PCa) in a subject, which comprises

(i) Determining the expression levels of the PCA3, PSMA and PSGR genes in a subject sample obtained at a first period of time and
(ii) Determining the expression levels of said genes PCA3, PSMA and PSGR genes in a sample of the same subject obtained at a second period of time, wherein said second period of time is later than said first period of time;
(iii) comparing the expression levels of said PCA3, PSMA and PSGR genes obtained at the first and at the second period of time with predetermined cut off values for each of said genes wherein said predetermined cut off values for each gene correspond to the expression level of said gene which correlates with the highest specificity at said desired sensitivity in a ROC (receiver operating characteristics) curves calculated based on the expression levels of the PCA3, PSMA and PSGR genes determined in a patient population being at risk of suffering prostate cancer,

wherein a significant decrease in expression levels of at least one of said genes in the subject sample at the second period of time with respect to said expression level at the first period of time is indicative of a positive progression of the subject
or
wherein a significant increase or a lack of change in expression levels of at least one of said genes in the subject sample at the second period of time with respect to said expression level at the first period of time is indicative of a negative progression of the subject.

6. A method according to claim 5

wherein steps (i) and (ii) further comprise the determination of PSAD in the patient and

wherein step (iii) further comprises comparing the PSAD level in the patient with a predetermined cut-off value for PSAD wherein said predetermined cut off value corresponds to a PSAD value which correlates with the highest specificity at said desired sensitivity in a ROC (receiver operating characteristics) curves calculated based on the expression levels of the PCA3, PSMA, PSGR genes and PSAD determined in a patient population being at risk of suffering prostate cancer, wherein a significant decrease in expression levels of at least one of said genes in the subject sample or of the PSAD value at the second period of time with respect to said expression level at the first period of time is indicative of a positive progression of the subject

or

wherein a significant increase or lack of change in expression levels of at least one of said genes in the subject sample or of the PSAD value at the second period of time with respect to said expression level at the first period of time is indicative of a negative progression of the subject.

7. A method for assessing if a subject has to be subjected to a prostate biopsy, said subject having a serum PSA range within 4-10 ng/mL, which comprises:

(i) determining the level of expression of genes PCA3, PSMA and PSGR in a sample isolated from said subject and

(ii) comparing the expression levels of said PCA3, PSMA and PSGR genes with predetermined cut off values for each of said genes wherein said predetermined cut off values for each gene correspond to the expression level of said gene which correlates with the highest specificity at said desired sensitivity in a ROC (receiver operating characteristics) curve calculated based on the expression levels of the PCA3, PSMA and PSGR genes determined in a patient population being at risk of suffering prostate cancer,

wherein a significant increase in the expression level of at least one of said genes in said sample with respect to said predetermined cut off value for said gene is indicative that the subject is candidate for prostate biopsy.

8. A method according to claim 7

wherein step (i) further comprises the determination of PSAD in the patient, wherein step (ii) further comprises comparing the PSAD level in the patient with a predetermined cut-off value for PSAD wherein said predetermined cut off value corresponds to a PSAD value which correlates with the highest specificity at said desired sensitivity in a ROC (receiver operating characteristics) curve calculated based on the expression levels of the PCA3, PSMA, PSGR genes and PSAD determined in a patient population being at risk of suffering prostate cancer and wherein increased expression level of at least one of said genes in said sample with respect to said predetermined cut off value for said gene or increased PSAD values with respect to said predetermined cut off value is indicative that the subject is candidate for prostate biopsy.

9. A method as defined in any of claims 1 to 8 wherein the patient population being at risk of suffering prostate cancer is formed by patients having PSA levels above 4 ng/mL and/or patients with a positive DRE.

10. A method according to claim 9 wherein the patient population being at risk of suffering prostate cancer is formed by patients having PSA levels lower than 10 ng/mL.

11. Method according to any of claims 1 to 10 wherein the sample is a sample of urine, prostatic secretions, ejaculation or blood.

12. Method according to any of claims 1 to 11 wherein the sample corresponds to sediments from voided urine samples obtained after prostate massage.

13. A method according to any of claims 1 to 12 wherein the desired sensitivity is at least 100%.

14. A method according to any of claims 1 to 13 wherein the expression levels of the PCA3, PSMA and PSGR genes are normalized to the expression level of the prostate housekeeping gene in the same sample wherein the PCA3, PSMA and PSGR are determined.

15. A method according to claim 14 wherein the prostate housekeeping gene is PSA.

16. A method according to any of claims 1 to 15 wherein the expression levels of the PCA3, PSMA and PSGR genes are determined by quantitative PCR.

17. A method according to any of claims 1 to 16 wherein the patient suffers isolated high-grade prostate intraepithelial neoplasia.

18. A kit comprising a first component and, optionally, a second component wherein the first component is a set of reagents consisting of:

   i) a reagent which allows determining the expression level of gene PCA3;
   ii) a reagent which allows determining the expression level of gene PSMA; and
   iii) a reagent which allows determining the expression level of gene PSGR; and

wherein the second component comprises consists of one or more reagents which allow the determination of the expression levels of one or more prostate housekeeping genes.

19. Use of a kit according to claim 18 for the diagnosis of PCa, for assessing or monitoring the response to therapy in a subject having PCa or for monitoring the progression of PCa in a subject.

20. Use according to claim 19 wherein the subject which is to be assessed as candidate for prostate biopsy is a subject having serum PSA levels of 4-10 ng/mL.

Fig. 1

Fig. 2

Fig. 3

Fig. 4

Europäisches
Patentamt

European
Patent Office

Office européen
des brevets

# EUROPEAN SEARCH REPORT

Application Number

EP 10 38 2194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2006 032394 A1 (UNIV DRESDEN TECH [DE]) 20 September 2007 (2007-09-20) | 1,3,5, 9-14, 16-20 | INV. C12Q1/68 |
| Y | * the whole document * | 2,4,6,15 | |
| A | | 7,8 | |
| | ----- | | |
| X | WO 02/081656 A2 (HENRY M JACKSON FOUNDATION [US]; GAO CHUN L [US]; MOUL JUDD W [US]; SR) 17 October 2002 (2002-10-17) | 1,3,5, 9-14, 16-20 | |
| Y | * claim 19 * | 2,4,6 | |
| A | | 7,8 | |
| | ----- | | |
| X | US 2004/241707 A1 (GAO CHUN L [US] ET AL) 2 December 2004 (2004-12-02) | 1,3,5, 9-14, 16-20 | |
| Y | * claim 19 * | 2,4,6,15 | |
| A | | 7,8 | |
| | ----- | | |
| X | FUSSEL S ET AL: "Extension of quantitative multi-gene expression studies on paired radical prostatectomy (RPE) - Prostate tissue samples" EUROPEAN UROLOGY SUPPLEMENTS, & 22ND ANNUAL CONGRESS OF THE EUROPEAN-ASSOCIATION-OF-UROLOGY; BERLIN, GERMANY; MARCH 21 -24, 2007, vol. 6, no. 2, 1 March 2007 (2007-03-01), page 190, XP002599910 | 1,3,5, 9-14, 16-20 | **TECHNICAL FIELDS SEARCHED (IPC)** C12Q |
| Y | * the whole document * | 2,4,6,15 | |
| A | | 7,8 | |
| | ----- | | |
| | -/-- | | |

The present search report has been drawn up for all claims

| Place of search | Date of completion of the search | Examiner |
|---|---|---|
| The Hague | 15 September 2010 | Franz, Cerstin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 10 38 2194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OHORI M ET AL: "Is prostate-specific antigen density more useful than prostate-specific antigen levels in the diagnosis of prostate cancer?" UROLOGY NOV 1995 LNKD- PUBMED:7495118, vol. 46, no. 5, November 1995 (1995-11), pages 666-671, XP002600865 ISSN: 0090-4295 | 7,8 | |
| Y | * the whole document * | 2,4,6 | |
| A | | 1,3,5, 9-20 | |
| A | GHOSH ARUNDHATI ET AL: "Novel role of prostate-specific membrane antigen in suppressing prostate cancer invasiveness." CANCER RESEARCH 1 FEB 2005 LNKD- PUBMED:15705868, vol. 65, no. 3, 1 February 2005 (2005-02-01), pages 727-731, XP002600866 ISSN: 0008-5472 * the whole document * | 1-20 | |
| A | SCHMIDT UTA ET AL: "Quantitative multi-gene expression profiling of primary prostate cancer." THE PROSTATE 1 OCT 2006 LNKD- PUBMED:16921506, vol. 66, no. 14, 1 October 2006 (2006-10-01), pages 1521-1534, XP002600867 ISSN: 0270-4137 * the whole document * | 1-20 | **TECHNICAL FIELDS SEARCHED (IPC)** |

-/--

The present search report has been drawn up for all claims

| Place of search | Date of completion of the search | Examiner |
|---|---|---|
| The Hague | 15 September 2010 | Franz, Cerstin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 407 555 A1**

Europäisches
Patentamt

European
Patent Office

Office européen
des brevets

# EUROPEAN SEARCH REPORT

Application Number

EP 10 38 2194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VENER TATIANA ET AL: "Development of a multiplexed urine assay for prostate cancer diagnosis." CLINICAL CHEMISTRY MAY 2008 LNKD-PUBMED:18339699, vol. 54, no. 5, May 2008 (2008-05), pages 874-882, XP002600868 ISSN: 0009-9147 * the whole document * ----- | 1-20 | |

TECHNICAL FIELDS
SEARCHED      (IPC)

The present search report has been drawn up for all claims

| Place of search | Date of completion of the search | Examiner |
|---|---|---|
| The Hague | 15 September 2010 | Franz, Cerstin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 38 2194

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2010

| Patent document cited in search report | Publication date | Patent family member(s) | Publication date |
|---|---|---|---|
| DE 102006032394 A1 | 20-09-2007 | NONE | |
| WO 02081656 A2 | 17-10-2002 | AU 2002245727 A1 | 21-10-2002 |
| US 2004241707 A1 | 02-12-2004 | NONE | |

For more details about this annex : see Official Journal of the European Patent Office, No. 12/82

## REFERENCES CITED IN THE DESCRIPTION

### Patent documents cited in the description

- WO 03009814 A
- DE 102006032394
- WO 2008121132 A, Further
- US 6054320 A
- WO 9824935 A

- WO 09135019 A
- WO 98045420 A
- WO 010235 A
- WO 2004070056 A
- WO 2005003387 A

### Non-patent literature cited in the description

- **JEMAL, A. et al.** *Cancer statistics, 2007. CA: a cancer journal for clinicians,* 2007, vol. 57, 43-66
- **LOEB, S. et al.** Exclusion of inflammation in the differential diagnosis of an elevated prostate-specific antigen (PSA. *Urol Oncol,* 2009, vol. 27, 64-66
- **PANNEK, J. ; PARTIN, A.W.** Prostate-specific antigen: what's new in 1997. *Oncology (Williston Park,* 1997, vol. 11, 1273-12781279-1282
- **CATALONA, WJ. et al.** Measurement of prostate-specific antigen in serum as a screening test for prostate cancer. *N Engl J Med,* 1991, vol. 324, 1156-61
- **MAKINEN, T. et al.** Second round results of the Finnish population-based prostate cancer screening trial. *Clin Cancer Res,* 2004, vol. 10, 2231-6
- **MARKS, L.S. ; BOSTWICK, D.G.** Prostate Cancer Specificity of PCA3 Gene Testing: Examples from Clinical Practice. *Rev Urol,* 2008, vol. 10, 175-181
- **SARAMAKI et al.** TMPRSS2:ERG fusion identifies a subgroup of prostate cancers with a favorable prognosis. *Clin Cancer Res,* 2008, vol. 14, 3395-3400
- **VENER et al.** Development of a multiplexed urine assay for prostate cancer diagnosis. *Clin Chem,* 2008, vol. 54, 874-882
- **LAXMAN et al.** *Cancer Res,* 2008, vol. 68, 645-649
- **VAN GILS MP et al.** *Clin Cancer Res,* 2007, vol. 13, 939-43
- **HESSELS D et al.** *Eur Urol,* 2003, vol. 44, 8-15
- **MARKS LS et al.** *Urology,* 2007, vol. 69, 532-5
- **GROSKOPF J et al.** *Clin Chem,* 2006, vol. 52, 1089-95
- **HAESE A et al.** *Eur Urol,* 2008, vol. 54, 1081-8
- **DOWDY ; WEARDEN.** Statistics for Research. John Wiley & Sons, 1983

- **PANG.** *Urology,* March 2004, vol. 63 (3), 609-12
- RNA Methodologies. A Laboratory Guide for Isolation and Characterization. Academic Press, 1998
- RNA Isolation and Characterization Protocols. Methods in Molecular Biology. Human Press, 1998, vol. 86, 143-151
- PCR Applications: protocols for functional genomics. Academic Press, 1999, 55-72
- Taqman™ PCR Reagent Kit, Protocol, part number 402823. *Applied Biosystems,* 1996
- **Y.S. LIE ; CJ. PETROPOLUS.** Advances in Quantitative PCR Technology: 5' Nuclease Assays. *Current Opinion in Biotechnology,* 1998, vol. 9, 43-48
- PCR applications: protocols for functional genomics. Rapid Thermal Cycling and PCR Kinetics. Academic Press, 1999, 211-229
- **SAMBROOK et al.** Molecular cloning: to Laboratory Manual. Cold Spring Harbor Laboratory Press, 2001, vol. 1-3
- **ELSASSER-BEILE, U. et al.** *Curr Drug Targets,* 2009, vol. 10, 118-25
- **ALTSCHUL S.F. et al.** Basic local alignment search tool. *J Mol Biol.,* 05 October 1990, vol. 215 (3), 403-10
- **HARTLEY et al.** *Radiology,* 1982, vol. 143, 29-36
- **DELONG et al.** *Biometrics,* 1988, vol. 44, 837-845
- **DOWDY ; WEARDEN.** Statistics for Research. John Wiley and Sons, 1983
- **RICHIE JP et al.** *Urology,* 1993, vol. 42, 365-74
- **CARVALHAL GF et al.** *J. Urol.,* 1999, vol. 161, 835-9
- *Trends in Genetics,* 2003, vol. 19, 362-365
- **SOKOLL et al.** *Urol. Clin. North Am.,* 1997, vol. 24, 253-259
- *Cancer Res.,* 1999, vol. 59, 5975-9
- **FAIR et al.** *Prostate,* 1997, vol. 32, 140-148

- **REITER et al.** *Proc. Natl. Acad. Sci. USA,* 1998, vol. 95, 1735-1740
- **LIN et al.** *Cancer Res.,* 1999, vol. 59, 4180-4184
- **OETTGEN et al.** *J. Biol. Chem.,* 2000, vol. 275, 1216-1225
- **HERNESS.** *Cancer Res.,* 2003, vol. 63, 329-336
- **BUSSEMAKERS et al.** *Cancer Res.,* 1999, vol. 59, 5975-5979
- **SRIKANTAN et al.** *Proc. Natl. Acad. Sci. USA,* 2000, vol. 97, 12216-12221
- **STOLK et al.** *Prostate,* 2004, vol. 60, 214-226
- **EL-SHIRBINY.** *Adv. Clin. Chem.,* 1994, vol. 31, 99
- **BRAWER et al.** *J. Urol.,* vol. 147, 841
- **LETRAN et al.** *J. Urol.,* 1998, vol. 160, 426
- **OHORI M et al.** *Urology,* 1995, vol. 46, 666-71
- **REMZI et al.** *J. Urol.,* 2005, vol. 174, 1256-60
- **BAKER, S. G.** *J Natl Cancer Inst,* 2003, vol. 95, 511-515
- **MARKS LS et al.** *Rev. Urol.,* 2008, vol. 10, 175-81
- **IWAKIRI J et al.** *J Urol,* 1993, vol. 149, 783-6
- **MENG FJ et al.** *Cancer Epidemiol Biomarkers Prev,* 2002, vol. 11, 305-9
- **PAIK S et al.** *N Engl J Med,* 2004, vol. 351, 2817-26
- **VAN DE VIJVER MJ et al.** *N. Engl. J. Med.,* 2002, vol. 347, 1999-2009
- **CERVERA DEVAL J et al.** *Actas Urol. Esp.,* 2004, vol. 28, 666-71
- **RABER M et al.** *Arch. Ital. Urol. Androl.,* 2000, vol. 72, 197-9
- **OUYANG B et al.** *J. Urol.,* 2009, vol. 181, 2508-132513-4